(19) Europäisches Patentamt
European Patent Office
Office européen des brevets

(11) EP 3 878 481 A1

(12) **EUROPEAN PATENT APPLICATION**

(43) Date of publication:
**15.09.2021 Bulletin 2021/37**

(51) Int Cl.:
***A61L 15/46*** *(2006.01)* ***A61L 15/60*** *(2006.01)*

(21) Application number: **20162736.1**

(22) Date of filing: **12.03.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Livinguard AG**
**6300 Zug (CH)**

(72) Inventor: **SWAMY, Sanjeev**
**6300 Cham/Zug (CH)**

(74) Representative: **Karl, Christof**
**Bardehle Pagenberg Partnerschaft mbB**
**Patentanwälte, Rechtsanwälte**
**Prinzregentenplatz 7**
**81675 München (DE)**

(54) **HYGIENE PRODUCT FOR REDUCING MICROBES AND/OR MALODORS**

(57) The present invention relates to a hygiene product like a diaper comprising a layer of a textile material to which one or more antimicrobial agents such as polydiallyldimethylammonium chloride (polyDADMAC) and/or one or more crosslinkers or binders such as cationic blocked isocyanate are adhered. The antimicrobial agents and crosslinkers prevent or inhibit, when the textile material comes into contact with urine, decomposition of urea, comprised by the urine, by urease.

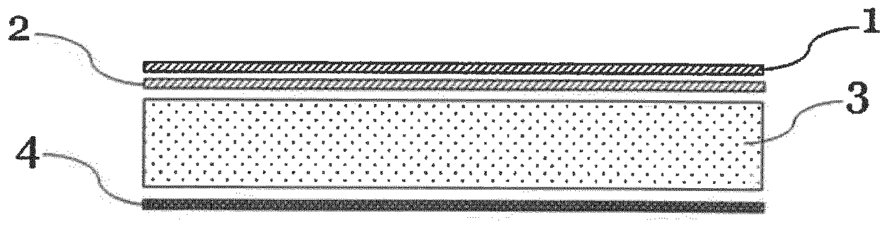


FIG. 1

EP 3 878 481 A1

**Description**

## FIELD OF THE INVENTION

The present invention relates to a textile material to which one or more agents are adhered which reduce microbes and/or reduce, when the textile comes into contact with body fluids such as urine, malodors from the body fluid. It also relates to a hygiene product such as diapers, adult incontinence products, tampons, and sanitary napkins, comprising the textile material according to the invention. Finally, it relates to a method of manufacturing a textile material according to the invention, as well as a use of a textile material according to the invention for reducing microbes and/or malodors caused by body fluids, in particular by urine.

## BACKGROUND OF THE INVENTION

Disposable hygiene products such as diapers, sanitary pads, tampons and incontinence products are usually worn over several hours. However, wearing disposable hygiene products over several hours may lead to undesired formation of odors and/or microbial infections, e.g. bacterial infections.

Due to the nature of body fluids such as blood and/or urine coming into contact with the disposable hygiene products, and the fact that they are next to a user's skin, bacteria can grow easily and cause skin irritation or other infections of a user. For example, diaper rash, TSS (toxic shock syndrome), urinary tract infections (UTIs) and related infections are caused by using disposable hygiene products for periods as long as 8 to 12 hours. Also, such infections can be easily passed on to nursing staff.

Furthermore, undesired odors may form from microbial decomposition of body fluids such as urine or blood. In particular the odor formed from decomposing urine is unpleasant and may be embarrassing. Urine odor is mainly caused by hydrolysis of urea to ammonia and carbon dioxide. Ammonia is a very strong-smelling compound. The hydrolysis of urea is an enzymatic reaction mediated by an enzyme called urease, which may be excreted by bacteria.

Textiles treated with antimicrobial agents are known in the art. However, in the context of disposable hygiene products, special requirements apply. For example, antimicrobial agents as well as the entire hygiene or personal care product need to comply with toxicity and safety requirements for human use. Also, undesired leaching of antimicrobial agents from an antimicrobially treated hygiene product or a textile material comprised by the hygiene product needs to be avoided. The provision of hygiene products comprising effective yet safe antimicrobial agents which are adhered to the hygiene product or a textile material comprised by the hygiene product is still a challenge.

Also, remedies for reducing or preventing the formation of odors from urine are known in the art, for example in the context of treating wastewater urine diversion systems or buildings for livestock or soil in agriculture. In this regard, urease inhibitors have been proposed, for reducing and/or preventing enzymatic urea decomposition by urease. For example, organic compounds such as phosphoramidates, hydroquinones, quinones, (di)substitutedthioureas, benzothiazoles, coumarin and phenolic aldehyde derivatives, and vanadium hydrazine complexes, together with B, Cu, S, Zn, ammonium thiosulfate, silver nanoparticles have been presented as urease inhibitors in the past (cf., for example, Modolo et al., Journal of Advanced Research 13, 2018, p. 29-37.). However, textile materials and/or disposable hygiene products known in the art do not allow for reducing or preventing odor formation from urine and/or other body fluids.

It is therefore an object of the invention to address one or more or all of the above-mentioned shortcomings of the prior art. Against this background, it is desirable to provide textile materials and/or hygiene products comprising textile materials allowing for both compliance with toxicity and safety requirements in human use and effectively preventing microbial infections and/or reducing or preventing odor formation from urine or other body fluids.

Some or all of these objects are achieved by the subject matter of the independent claims. Preferred embodiments are subject of the dependent claims.

## SUMMARY OF THE INVENTION

Generally, the present invention relates to a textile material to which one or more agents are adhered which reduce microbes, or which reduce, when the textile comes into contact with body fluids, in particular urine, malodors from the body fluid. Such a textile material can be used in hygiene products, for example diapers, adult incontinence products, bed pads, underwear, and sanitary napkins.

Apart from the fact that bacteria are inactivated by a textile material of the present invention, the inventors have surprisingly found that urease, i.e. the enzyme decomposing urea and thus causing malodors from urine, is inhibited when urine comes in contact with the treated textile material. Therefore, the present invention may provide for superior antimicrobial efficiency as well as superior reduction and/or prevention of odors from urine.

A 1st aspect of the invention is directed to a textile material to which one or more agents are adhered which reduce, when the textile comes into contact with urine, malodors from the urine.

A 2nd aspect of the invention is directed to a textile material to which one or more agents are adhered, preferably according to the preceding aspect, wherein the one or more agents prevent or inhibit, when the textile comes into contact with urine, decomposition of urea, comprised by the urine, by urease.

Acording to a 3rd aspect, in the textile material according to the preceding aspect, the decomposition is reduced, in comparison to the decomposition of urea in urine which does not come into contact with the textile material, at least by 20%, preferably at least by 40%, more preferably at least by 60%, even more preferably at least by 70%, and most preferably at least by 80%.

According to a 4th aspect, in the textile material according to the preceding aspect, the reduction is achieved within 0.5 to 300 minutes, preferably within 1 to 200 minutes, more preferably within 5 to 180 minutes, even more preferably within 10 to 160 minutes, even more preferably within 15 to 150 minutes.

According to a 5th aspect, in the textile material according to any one of 2nd to 4th aspects, the decomposition of urea is determined by conductivity measurements.

According to a 6th aspect, in the textile material of any one of the preceding aspects, the one or more agents comprise polyelectrolytes, preferably synthetic polyelectrolytes, more preferably cationic and/or hydrophilic polyelectrolytes, even more preferably cationic and/or hydrophilic polyquaterniums, most preferably polyDADMAC.

A 7th aspect of the invention is directed to a textile material to which one or more antimicrobial agents and/or one or more crosslinkers or binders are adhered.

According to an 8th aspect, in the textile material according to the preceding aspect, the textile material is a textile material according to any one of the 1st to 6th aspect.

According to a 9th aspect, in the textile material according to the preceding aspect, when the textile comes into contact with urine, the one or more antimicrobial agents and/or the one or more crosslinkers or binders reduce malodors from the urine and/or prevent or inhibit decomposition of urea, comprised by the urine, by urease.

According to a 10th aspect, in the textile material of any one of the 7th to 9th aspects, one or more or preferably all of the crosslinkers or binders and/or antimicrobial agents are not hydrophobic, preferably hydrophilic.

According to an 11th aspect, in the textile material of any one of 7th to 10th aspects, one or more or preferably all of the crosslinkers or binders and/or antimicrobial agents are cationic or non-ionic, preferably cationic.

According to a 12th aspect, in the textile material of any one of the 7th to 11th aspects, the one or more crosslinkers or binders are an isocyanate, preferably a blocked isocyanate, more preferably a cationic blocked isocyanate, most preferably Meikanate.

According to a 13th aspect, in the textile material of any one of the 7th to 12th aspects, the one or more crosslinkers are provided in a composition comprising water, emulsifier, and a blocked isocyanate.

According to a 14th aspect, in the textile material of the preceding aspect, the blocked isocyanate is made from the terminating group A according to structural formula (1)

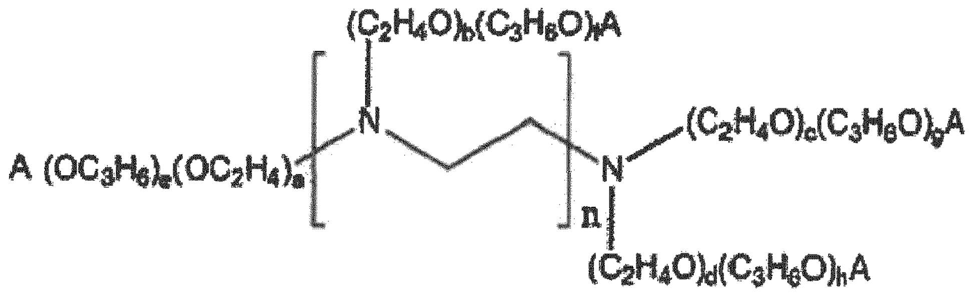

and the building blocks according to the following structural formulae (2) and (3)

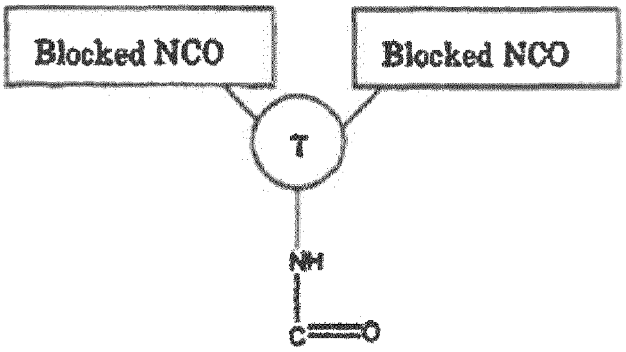

(2),

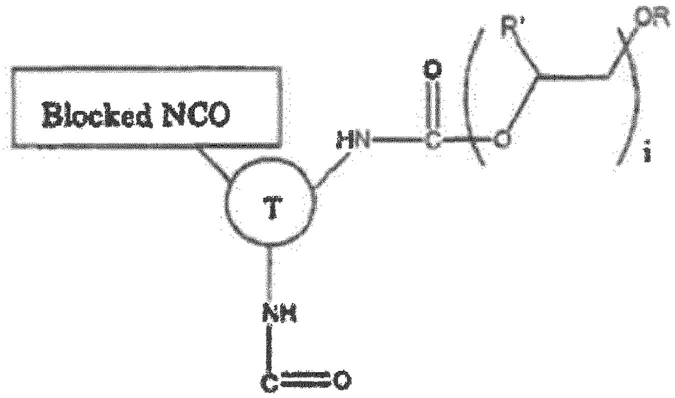

(3),

wherein in structural formulae (1) to (3) R represents an alkyl group, R' represents a hydrogen atom or a methyl group, Blocked NCO represents a group in which an isocyanate group is blocked by a blocking agent, and T represents an isocyanate type or adduct type triisocyanate skeleton, and n = 1-5, a, b, c, d = 0-4, e, f, g, h = 0-4, a + e, b + f, c + g, d + h = 1-4, i = 4 - 50, and
wherein the ratio of the building blocks (1) to (3) may vary.

According to a 15th aspect, in the textile material of preceding aspect, the blocked isocyanate comprises the terminating group A according to structural formula (1) in a range of 59 to 88%, the building block according to structural formula (2) in a range of 2 to 14% and the building block according to structural formula (3) in a range of 10 to 30%, and wherein the sum of terminating group A according to structural formula (1) and the building block according to structural formula (2) is in the range of 70-90% based on the total molecular weight of the blocked isocyanate.

According to a 16th aspect, in the textile material of any one of the 14th or 13th aspects, the triisocyanate compound is a trimethylolpropane adduct of tolylene diisocyanate.

According to a 17th aspect, in the textile material according to any one of the 7th to 16th aspects, the one or more antimicrobial agents are one or more selected from the group consisting of polyglucosamine (chitosan), arginine, polydiallyldimethylammonium chloride (polyDADMAC), and any combination thereof, preferably polyglucosamine (chitosan), and/or polydiallyldimethylammonium chloride (polyDADMAC).

According to an 18th aspect, in the textile according to any one of the 7th to 17th aspects, the one or more antimicrobial agents and/or the treated textile material substantially do not comprise silver and/or any other metals.

According to a 19th aspect, in the textile material of any one of the 7th to 18th aspects, one or more or preferably all of the crosslinkers or binders and/or of the antimicrobial agents are permanently adhered to the textile material.

According to a 20th aspect, in the textile material of any one of the preceding, aspects, the textile material comprises at least one acid selected from the group consisting of acetic acid, citric acid, salicylic acid, benzoic acid and sorbic acid, or salts thereof, in particular sodium salts thereof, preferably citric or acetic acid, most preferably citric acid.

According to a 21st aspect, in the textile material of any one of the preceding aspects, the textile material comprises a hydrophilic agent, preferably a hydrophilic agent enhancing the horizontal wicking rate according to AATCC test method 198-2013 and/or the absorbency time according to AATCC test method 79-2014 when compared to a textile material not comprising the hydrophilic agent, more preferably polyethylene glycol (PEG) selected from PEG-400, PEG-500, PEG-600 and/or PEG-800, most preferably PEG-400.

According to a 22nd aspect, in the textile material of any one of the preceding aspects, the textile material comprises a fabric softener, preferably a silicone, more preferably a functionalized silicone, even more preferably an amino-functionalized silicone, most preferably Ultratex®.

According to a 23rd aspect, in the textile material of any one of the preceding aspects, the textile material is a fiber, a yarn, or a fabric, preferably a fabric.

According to a 24th aspect, in the textile material of the preceding aspect, the textile material is selected from the group consisting of woven, knitted, warp-knitted, crocheted fabrics.

According to a 25th aspect, in the textile material of the 23rd aspect, the textile material is a non-woven fabric, such as a bonded or spun bonded or melt blown or hot melted fabric, and any combination thereof.

According to a 26th aspect, in the textile material of any one of the preceding aspects, the textile material is a cellulosic material, a synthetic material or a blend of cellulosic and synthetic material, preferably a synthetic material.

According to a 27th aspect, in the textile material of the preceding aspect, the cellulosic material comprises cotton, viscose, rayon, linen, hemp, ramie, jute, or combinations (blends) thereof, preferably viscose.

According to a 28th aspect, in the textile material of any one of the 26th or 27th aspects, the synthetic material comprises one or more selected from the group consisting of polyester, polyamide (nylon), acrylic polyester, spandex (elastane, Lycra), aramids, modal, sulfar, polylactide (PLA), lyocell, polybutyl tetrachloride (PBT), polypropylene (PP), and combinations (blends) thereof, preferably polypropylene (PP).

According to a 29th aspect, the textile material of any one of the preceding aspects comprises polydiallyldimethylammonium chloride (polyDADMAC) in a relative amount per weight based on weight of fabric of at least 1 wt.-%, preferably of at least 2 wt.-%, more preferably of at least 4 wt.-%, even more preferably of at least 8 wt.-%, most preferably of at least 11 wt.-% and/or of at most 50 wt.-%, preferably of at most 30 wt.-%, more preferably of at most 20 wt.-%, even more preferably of at most 15 wt.-%, most preferably of at most 12 wt.-%.

According to a 30th aspect, the textile material of any one of the preceding aspects comprises polyglucosamine (chitosan) in a relative amount per weight based on weight of fabric of at least 0.05 wt.-%, preferably of at least 0.1 wt.-%, more preferably of at least 0.2 wt.-%, even more preferably of at least 0.3 wt.-%, most preferably of at least 0.4 wt.-% and/or of at most 1.5 wt.-%, preferably of at most 1.0 wt.-%, more preferably of at most 0.8 wt.-%, even more preferably of at most 0.6 wt.-%, most preferably of at most 0.4 wt.-%.

According to a 31st aspect, the textile material of any one of the preceding aspects comprises cationic blocked isocyanate, preferably Meikanate, in a relative amount per weight based on weight of fabric of at least 1 wt.-%, preferably of at least 2 wt.-%, more preferably of at least 4 wt.-%, even more preferably of at least 6 wt.-%, most preferably of at least 8 wt.-% and/or of at most 30 wt.-%, preferably of at most 20 wt.-%, more preferably of at most 15 wt.-%, even more preferably of at most 12 wt.-%, most preferably of at most 8 wt.-%.

According to a 32nd aspect, the textile material of any one of the preceding aspects comprises acid, preferably citric acid, in a relative amount per weight based on weight of fabric of at least 0.01 wt.-%, preferably of at least 0.03 wt.-%, more preferably of at least 0.06 wt.-%, even more preferably of at least 0.08 wt.-%, most preferably of at least 0.12 wt.-% and/or of at most 1 wt.-%, preferably of at most 0.5 wt.-%, more preferably of at most 0.2 wt.-%, even more preferably of at most 0.15 wt.-%, most preferably of at most 0.12 wt.-%.

According to a 33rd aspect, the textile material of any one of the preceding aspects comprises arginine, in a relative amount per weight based on weight of fabric of at least 1 wt.-%, preferably of at least 2 wt.-%, more preferably of at least 4 wt.-%, even more preferably of at least 6 wt.-%, most preferably of at least 8 wt.-% and/or of at most 30 wt.-%, preferably of at most 20 wt.-%, more preferably of at most 15 wt.-%, even more preferably of at most 12 wt.-%, most preferably of at most 8 wt.-%.

According to a 34th aspect, the textile material of any one of the preceding aspects comprises a hydrophilic agent, preferably polyethylene glycol (PEG) selected from PEG-400, PEG-500, PEG-600 and/or PEG-800, most preferably PEG-400, preferably PEG-400, in a relative amount per weight based on weight of fabric of at least 2 wt.-%, preferably of at least 4 wt.-%, more preferably of at least 8 wt.-%, even more preferably of at least 12 wt.-%, most preferably of at least 16 wt.-% and/or of at most 60 wt.-%, preferably of at most 40 wt.-%, more preferably of at most 30 wt.-%, even more preferably of at most 24 wt.-%, most preferably of at most 16 wt.-%.

A 35th aspect of the invention is directed to a method of manufacturing a textile material according to any one of the 1st to 34th aspects, comprising the steps of

- treating a textile material using a liquor application process, preferably a padding process,
- preferably heat-treating the treated textile material at a temperature of at least 80 °C, preferably at least 100 °C, more preferably at least 110 °C, and most preferably at least about 120 °C and/or at a heat-treatment temperature of at most 160 °C, preferably at most 140 °C, more preferably at most 130 °C, and most preferably at most about 120 °C,
- wherein heat-treatment is preferably conducted for a time period of at least 60 seconds, preferably at least 90 seconds, more preferably at least 120 seconds, even more preferably at least 180 seconds, and most preferably

for at least 240 seconds and/or for a heat treatment time of at most 600 seconds, preferably at most 500 seconds, more preferably at most 400 seconds, even more preferably at most 300 seconds, and most preferably at most 240 seconds.

According to a 36th aspect, in the method of the preceding aspect, the liquor of the liquor application process comprises one or more or all of the agents adhered to the textile material.

According to a 37th aspect, in the method of any one the 35th or 36th aspects, the liquor of the liquor application process comprises chitosan in an amount of at least 1 $g \cdot L^{-1}$, preferably at least 5 $g \cdot L^{-1}$, more preferably at least 10 $g \cdot L^{-1}$, even more preferably at least 15 $g \cdot L^{-1}$, and most preferably at least 20 $g \cdot L^{-1}$ and/or in an amount of at most 100 $g \cdot L^{-1}$, preferably at most 60 $g \cdot L^{-1}$, more preferably at most 40 $g \cdot L^{-1}$, even more preferably at most 30 $g \cdot L^{-1}$, and most preferably at most 20 $g \cdot L^{-1}$.

According to a 38th aspect, in the method of any one of the 35th to 37th aspects, the liquor of the liquor application process comprises polyDADMAC in an amount of at least 5 $g \cdot L^{-1}$, preferably at least 10 $g \cdot L^{-1}$, more preferably at least 20 $g \cdot L^{-1}$, even more preferably at least 30 $g \cdot L^{-1}$, and most preferably at least 40 $g \cdot L^{-1}$ and/or in an amount of at most 1000 $g \cdot L^{-1}$, preferably at most 300 $g \cdot L^{-1}$, more preferably at most 200 $g \cdot L^{-1}$, even more preferably at most 150 $g \cdot L^{-1}$, and most preferably at most 100 $g \cdot L^{-1}$.

According to a 39th aspect, in the method of any one of the the 35th to 38th aspects, the liquor of the liquor application process comprises BKC in an amount of at least 1 $g \cdot L^{-1}$, preferably at least 2 $g \cdot L^{-1}$, more preferably at least 4 $g \cdot L^{-1}$, even more preferably at least 6 $g \cdot L^{-1}$, and most preferably at least 10 $g \cdot L^{-1}$ and/or in an amount of at most 60 $g \cdot L^{-1}$, preferably at most 40 $g \cdot L^{-1}$, more preferably at most 30 $g \cdot L^{-1}$, even more preferably at most 20 $g \cdot L^{-1}$, and most preferably at most 10 $g \cdot L^{-1}$.

According to a 40th aspect, in the method of any one of the 35th to 39th aspects, the liquor of the liquor application process comprises Meikanate in an amount of at least 1 $g \cdot L^{-1}$, preferably at least 5 $g \cdot L^{-1}$, more preferably at least 10 $g \cdot L^{-1}$, even more preferably at least 15 $g \cdot L^{-1}$, and most preferably at least 20 $g \cdot L^{-1}$ and/or in an amount of at most 100 $g \cdot L^{-1}$, preferably at most 60 $g \cdot L^{-1}$, more preferably at most 40 $g \cdot L^{-1}$, even more preferably at most 30 $g \cdot L^{-1}$, and most preferably at most 20 $g \cdot L^{-1}$.

According to a 41st aspect, in the method of any one of the 35th to 40th aspects, the liquor of the liquor application process comprises citric acid or acetic acid in an amount of at least 0.05 $g \cdot L^{-1}$, preferably at least 0.1 $g \cdot L^{-1}$, more preferably at least 0.15 $g \cdot L^{-1}$, even more preferably at least 0.2 $g \cdot L^{-1}$, and most preferably at least 0.3 $g \cdot L^{-1}$ and/or in an amount of at most 1 $g \cdot L^{-1}$, preferably at most 0.7 $g \cdot L^{-1}$, more preferably at most 0.5 $g \cdot L^{-1}$, even more preferably at most 0.4 $g \cdot L^{-1}$, and most preferably at most 0.3 $g \cdot L^{-1}$.

According to a 42nd aspect, in the method of any one of the 35th to 41th aspects, the liquor of the liquor application process comprises PEG-400 in an amount of at least 10 $g \cdot L^{-1}$, preferably at least 20 $g \cdot L^{-1}$, more preferably at least 30 $g \cdot L^{-1}$, even more preferably at least 40 $g \cdot L^{-1}$, and most preferably at least 50 $g \cdot L^{-1}$ and/or in an amount of at most 200 $g \cdot L^{-1}$, preferably at most 150 $g \cdot L^{-1}$, more preferably at most 100 $g \cdot L^{-1}$, even more preferably at most 75 $g \cdot L^{-1}$, and most preferably at most 50 $g \cdot L^{-1}$.

A 43th aspect of the invention is directed to a textile obtainable according to any one of the 35th to 42nd aspects.

A 44th aspect of the invention is directed to a hygiene product comprising a first layer comprising a textile material according to any one of the 1st to 35th or 43th aspects, and preferably a second layer comprising a textile material according to any one of the 1st to 35th or 43th aspects.

According to a 45th aspect, in the hygiene product of the preceding aspect, the hygiene product is a diaper, adult incontinence product, bed pad, underwear liner, or sanitary napkins.

According to a 46th aspect, in the hygiene product of any one of the 44th or 45th aspects, the hygiene product is configured such that during ordinary use of the hygiene product, the first layer is closer to the body of the user than the second layer.

According to a 47th aspect, in the hygiene product of any one of the 44th to 46th aspects, the first layer and/or the second layer are or comprise treated polypropylene fabrics.

According to a 48th aspect, the hygiene product of any one of the 44th to 47th aspects comprises an additional layer of absorber material.

According to a 49th aspect, in the hygiene product of the preceding aspect, the hygiene product is configured such that during ordinary use of the hygiene product, the first layer and where existent the second layer are closer to the body of the user than the layer of absorber material.

According to a 50th aspect, in the hygiene product of any one of the 48th or 49th aspect, the layer of absorber material is in direct contact with the first layer or the second layer.

According to a 51st aspect, in the hygiene product of any one of the 48th to 50th aspects, the absorber material comprises one or more of superabsorbent polymers (SAPs).

According to a 52nd aspect, in the hygiene product of the preceding aspect, the layer of absorber material does not comprise a textile material according to any one of the 1st to 34th or 43rd aspects.

According to a 53rd aspect, the hygiene product of any one of the 44th to 52nd aspects comprises a water-impermeable layer.

According to a 54th aspect, in the hygiene product of the preceding aspect, the hygiene product is configured such that during ordinary use of the hygiene product, the first layer and where existent the second layer and layer of absorber material are closer to the body of the user than the water-impermeable layer.

According to a 55th aspect, in the hygiene product of any one of the 53rd or 54th aspects, the water-impermeable layer comprises a polyethylene (PE) film.

A 56th aspect of the invention is directed to a method of killing or reducing a number of microbes with a textile material of any one of the 1st to 34th or 43th aspects, or use of a textile material of any one of 1st to 34th or 43th aspects for killing or reducing a number of microbes, comprising bringing the microbes and/or a liquid comprising the microbes into contact with the textile material.

According to a 57th aspect, in the method or use of the preceding aspect, the liquid is urine or blood.

According to a 58th aspect, in the method or use of any one of the 56th or 57th aspects, the microbes are bacteria, virus, or fungi, preferably bacteria.

According to a 59th aspect, in the method or use of any one of the 56th to 58th aspects, the liquid comprising the microbes is passed through a layer comprising the textile material.

A 60th aspect of the invention is directed to a method of reducing malodors of urine with a textile material according to any one of the 1st to 34th or 43rd aspects, or use of a textile material of any one of the 1st to 34th or 43rd aspects for reducing malodors of urine, comprising bringing the urine in contact with the textile material.

According to a 61st aspect, in the method or use according to the preceding aspect, the urine is passed through a layer comprising the textile material.

According to a 62nd aspect, in the method or use of the preceding aspect, the method or use is a method or use according to any one of the 56th to 59th aspects.

According to a 63rd aspect, in the method or use according to any one of the 56th to 62th aspects, the textile material is comprised by a hygiene product of any one of the 44th to 55th aspects.

According to a 64th aspect, in the method or use of the preceding aspect, the method or use is carried out while a human user is wearing the hygiene product.

## BRIEF DESCRIPTION OF THE DRAWING

**Fig. 1** shows a schematic representation of a hygiene product according to the present invention, for example a diaper or adult incontinence product, comprising several layers.

## DETAILED DESCRIPTION OF THE INVENTION

### *Definitions*

The term *"antimicrobial"* as used in the context of the present invention relates to the ability to act against at least one or more types of microorganisms, e.g. by killing microorganisms, or to inhibit the growth or reproduction of at least one or more types of microorganisms, such as viruses, fungi or bacteria. If specifically, viruses are targeted, then it may also be referred to as *"antiviral"* or *"virucidal",* in case of fungi *"antifungal"* or *"fungicidal",* or in case of bacteria *"antibacterial"* or *"bactericidal"* ability. In the sense of the present invention, the ability to act against at least one or more types of microorganisms (i.e. *"antimicrobial"* or *"microbiocidal"* ability), the ability to act against at least one or more types of bacteria (i.e. *"antibacterial"* or *"bactericidal"* ability), the ability to act against at least one or more types of viruses (i.e. *"antiviral"* or *"virucidal"* ability), or the ability to act against at least one or more types of fungi (i.e. *"antifungal"* or *"fungicidal"* ability), refer to the ability of reducing the number of microorganisms, or bacteria, viruses or fungi, respectively by a log reduction of at least 0.5, preferably of at least 1, more preferably of at least 2, most preferably of at least 3 within 24 hours, preferably within 12 hours, more preferably within 8 hours, even more preferably within 4 hours, and most preferably within 10 minutes.

The term *"antimicrobial agent"* as used in the context of the present invention relates to a chemical compound that acts antimicrobially against at least one or more types of microorganisms, for example when the agent is applied to a substrate, and the substrate comes into contact with the microorganisms. Said term relates to any compound, agent, product or composition that is harmful to one or more "microorganism" as used in the context of the present invention. Preferably, the one or more "microorganisms" get killed by the "antimicrobial" product or process. Exemplary antimicrobial agents are polyglucosamine (chitosan), arginine, or polydiallyldimethylammonium chloride (polyDADMAC).

The term *"crosslinker"* or *"binder"* as used in the context of the present invention relates to chemical agents

allowing for forming crosslinks, in particular covalent bonds and/or relatively short sequences of chemical bonds to join two molecules, for example polymers together. Crosslinks can be formed by chemical reactions that are, for example, initiated by heat, pressure, change in pH, or irradiation.

The term *"hydrophilic"* as used in the context of the present invention relates to a physical property of a molecule or portion of a molecule. Specifically, interactions of hydrophilic molecules or portions thereof with water and other polar substances are more thermodynamically favorable than their interactions with oil or other hydrophobic solvents. In case a hydrophilic agent is comprised by a textile material, it increases the hydrophilicity of the textile material compared to same textile material not treated with the agent. E.g. the textile material treated with a hydrophilic agent has a higher water absorbency, or an increased horizontal or vertical wicking rate when compared to the corresponding untreated textile material. Consequently, an agent comprised by a textile material is *"not hydrophilic"* if it does not increase the hydrophilicity of the textile material compared to the same textile material not treated with the agent. The term *"hydrophobic"* relates to a physical property of a molecule or portion thereof. Specifically, hydrophilic molecules or portions thereof are repelled from water. An agent comprised by a textile material is *"hydrophobic"* if it decreases the hydrophilicity of the textile material compared to the same textile material not treated with the agent.

The term *"cationic"* as used in the context of the present invention relates to the presence of positive electric charges in or on a material. The term *"anionic"* relates to the presence of negative electric charges in or on a material. The term *"non-ionic"* relates to substantially absence of electric charges. An agent comprised by a textile material is *"cationic"* if the textile material compared to the same textile material not treated with the agent is more cationic or less anionic. An agent comprised by a textile material is *"anionic"* if the textile material compared to the same textile material not treated with the agent is more anionic or less cationic. An agent comprised by a textile material is *"non-ionic"* if the textile material compared to the same textile material not treated with the agent is substantially nor more or less anionic or cationic.

The term *"textile material"* as used herein means a textile material in any form and includes fibers, yarns, threads, ply yarns, fabrics produced from fibers and/or yarns, and the finished products produced from fibers, yarns, and/or fabrics. The textile material can be woven, knitted, crocheted, bonded and/or non-woven fabric. It can be spun, electrospun, drawn, meltblown or extruded, or a combination of these

The terms *"adhere to"* or *"adherence"* as used in the context of the present invention relates to any physical or chemical interaction between one or more antimicrobial agents as used in the present invention and the textile material, a fabric or fibers comprised by the textile material. Such physical or chemical interaction may include, but are not limited to, adsorption, absorption, ionic binding, non-ionic binding, covalent binding and/or binding via weak interaction forces such as van-der-Waals forces or Hydrogen-bonds.

Whenever a temperature is mentioned in the present specification, the temperature refers to a temperature applied at normal pressure (101.325 Pa). If in an implementation of the invention higher or lower pressure is applied, the temperatures are understood to be adapted accordingly.

All percentages hereinafter refer to weight percentage unless otherwise defined.

***Main aspects of the present invention***

Preferred embodiments and examples of the present invention will be described in the following detailed description. It is emphasized that the present invention is not limited to these embodiments.

A first aspect of the present invention is directed to textile material to which one or more agents are adhered which reduce, when the textile comes into contact with urine, malodors from the urine. Another aspect of the present invention relates to a textile material to which one or more agents are adhered, wherein the one or more agents prevent or inhibit, when the textile comes into contact with urine, decomposition of urea, comprised by the urine, by urease. This way, the formation of undesired, unpleasant and embarrassing odors can effectively be reduced and/or prevented.

According to a preferred embodiment of the invention, the decomposition of urea is reduced, in comparison to the decomposition of urea in urine which does not come into contact with the textile material of the invention, at least by 20%, preferably at least by 40%, more preferably at least by 60%, even more preferably at least by 70%, and most preferably at least by 80% and/or the reduction is achieved within 0.5 to 300 minutes, preferably within 1 to 200 minutes, more preferably within 5 to 180 minutes, even more preferably within 10 to 160 minutes, even more preferably within 15 to 150 minutes. Preferably, the decomposition of urea is determined by conductivity measurements.

Another aspect of the invention is directed to a textile material comprising one or more antimicrobial agents and/or one or more crosslinkers or binders. The textile material provides for an antimicrobial effect, and the antimicrobial agents are stably adhered to the textile material. When the textile comes into contact with urine, the one or more antimicrobial agents and/or the one or more crosslinkers or binders preferably reduce malodors from the urine and/or prevent or inhibit decomposition of urea, comprised by the urine, by urease.

The textile material of the present invention preferably exhibits a reduction value of *Escherichia coli* and/or *Staphylococcus aureus,* measured in accordance with AATCC test method 100-1999 and/or AATCC test method

100-2012, of at least 90%, preferably at least 99%, more preferably at least 99.9%, most preferably at least 99.99%, within 24 hours of contact time.

The inventors believe that using the textile material of the present invention, bacteria can are killed or inactivated, in particular bacteria which excrete enzymes which cause hydrolysis of urea to ammonia and carbon dioxide. Furthermore, they believe that the hydrolytic enzyme is inhibited by the textile material of the invention. Concluding, it seems that multiple mechanisms (bacterial inactivation and enzyme inhibition) help in the odor prevention.

***Preferred antimicrobial agents***

A great variety of antimicrobial agents can be used in producing the textile material according to the present invention.

The antimicrobial agents are preferably cationic, or at least non-ionic, i.e. neutral, but not anionic. The inventors found that anionic compounds do not bind well to cellulosic fibers subjected to treatment with the antimicrobial agents of the present invention and can easily be removed, e.g. by salts. Cationic (acid) agents are believed to attack the textile fibers and thereby adhere to them. It is believed that the killing mechanism of cationic agents is based on disrupting membranes of the microbes, allowing the free exchange of intra- and extra-cellular ions.

The antimicrobial agents may be hydrophobic, but preferably they are hydrophilic, or at least not hydrophobic. Non-hydrophobic, in particular hydrophilic agents are preferred because they support absorbency of body fluids by and/or distribution of body fluids in the textile material, or at least they do not counter the absorbency or distribution.

The one or more antimicrobial agents may comprise or consist of polyelectrolytes, preferably synthetic polyelectrolytes, more preferably cationic and/or hydrophilic polyelectrolytes, even more preferably cationic and/or hydrophilic polyquaterniums, most preferably polydiallyldimethylammonium chloride (polyDADMAC). The inventors found that when applied to textile materials, these agents surprisingly are efficient antimicrobial agents. They also comply with high toxicity and safety standards, which makes them specifically suitable for use in consumer products, preferably hygiene or personal care products, in particular disposable hygiene products.

PolyDADMAC is known in the art and commercially available. It belongs to the cationic and hydrophilic quaternary ammonium compounds (polyquats) and has the structural formula as shown below, wherein n indicates the number of the repeating units:

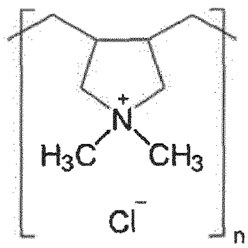

In a preferred embodiment of the invention, the textile material comprises polydiallyldimethylammonium chloride (polyDADMAC) in a relative amount per weight based on weight of fabric of at least 1 wt.-%, preferably of at least 2 wt.-%, more preferably of at least 4 wt.-%, even more preferably of at least 8 wt.-%, most preferably of at least 11 wt.-% and/or of at most 50 wt.-%, preferably of at most 30 wt.-%, more preferably of at most 20 wt.-%, even more preferably of at most 15 wt.-%, most preferably of at most 12 wt.-%.

The one or more antimicrobial agents may comprise or consists of chitosan. Chitosan is known in the art and commercially available. Chitosan is also an antimicrobial agent complying with high toxicity and safety standards, which makes it specifically suitable for use in consumer products, preferably hygiene or personal care products, in particular disposable hygiene products. Chitosan is a linear polysaccharide composed of randomly distributed β-(1→4)-linked D-glucosamine (deacetylated unit) and N-acetyl-D-glucosamine (acetylated unit) and has the structural formula as shown hereinafter, wherein n indicates the number of the monomer units:

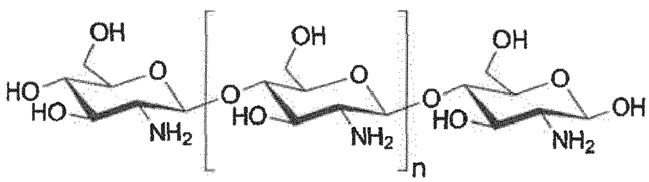

Chitosan may have any molecular weight known in the art, i.e. low molecular weight such as 50,000-190,000

Da, medium molecular weight such as 190,000-310,000 Da and/or high molecular weight such as 310,000-3750,00 Da.

In a preferred embodiment of the invention, the textile material comprises chitosan in a relative amount per weight based on weight of fabric of at least 0.05 wt.-%, preferably of at least 0.1 wt.-%, more preferably of at least 0.2 wt.-%, even more preferably of at least 0.3 wt.-%, most preferably of at least 0.4 wt.-% and/or of at most 1.5 wt.-%, preferably of at most 1.0 wt.-%, more preferably of at most 0.8 wt.-%, even more preferably of at most 0.6 wt.-%, most preferably of at most 0.4 wt.-%.

The one or more antimicrobial agents may comprise or consist of arginine. Arginine is an α-amino acid known in the art and commercially available. Arginine has a structure as shown hereinafter:

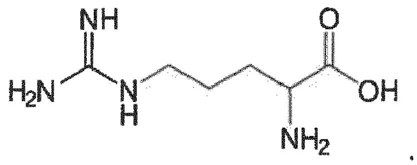

In a preferred embodiment of the invention, the textile material comprises arginine in a relative amount per weight based on weight of fabric of at least 1 wt.-%, preferably of at least 2 wt.-%, more preferably of at least 4 wt.-%, even more preferably of at least 6 wt.-%, most preferably of at least 8 wt.-% and/or of at most 30 wt.-%, preferably of at most 20 wt.-%, more preferably of at most 15 wt.-%, even more preferably of at most 12 wt.-%, most preferably of at most 8 wt.-%.

In the preferred embodiments of the present invention, one or more or all of the one or more antimicrobial agents are permanently adhered to the textile material. This way, the textile material of the present invention not only provides for an antimicrobial effect but also complies with toxicity and safety requirements for human use.

***Preferred crosslinkers or binders***

A great variety of crosslinkers or binders can be used in producing the textile material according to the present invention.

The crosslinkers or binders may be hydrophobic, but preferably they are hydrophilic, or at least not hydrophobic. Non-hydrophobic, in particular hydrophilic crosslinkers or binders are preferred because they support absorbency of body fluids by and/or distribution of body fluids in the textile material, or at least they do not counter the absorbency or distribution.

The crosslinkers or binders may comprise one or more cationic crosslinkers, anionic crosslinkers, or non-ionic crosslinkers, preferably cationic crosslinkers. Cationic crosslinkers or binders are preferred because they may have an antimicrobial effect, or at least not counter the effect of antimicrobial agents used in combination with the crosslinkers or binders, which antimicrobial agents are mostly cationic.

In preferred embodiments, the one or more crosslinkers or binders are an isocyanate, preferably a blocked isocyanate, more preferably a cationic blocked isocyanate, most preferably Meikanate. The inventors found that these crosslinkers or binders allow for stable adherence of the one or more agents to the textile material, and the textile material also complies with toxicity and safety requirements for human use.

For example, the one or more crosslinkers may be provided in a composition comprising water, emulsifier, and a blocked isocyanate. The blocked isocyanate may be made from the terminating group A according to the following structural formula (1):

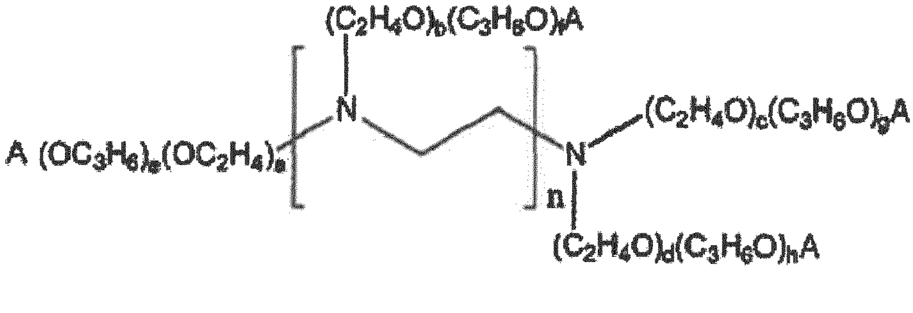

(1)

The building blocks may be made according to the following structural formulae (2) and (3):

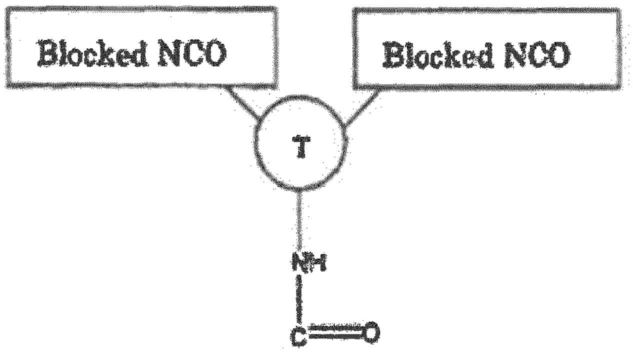

(2)

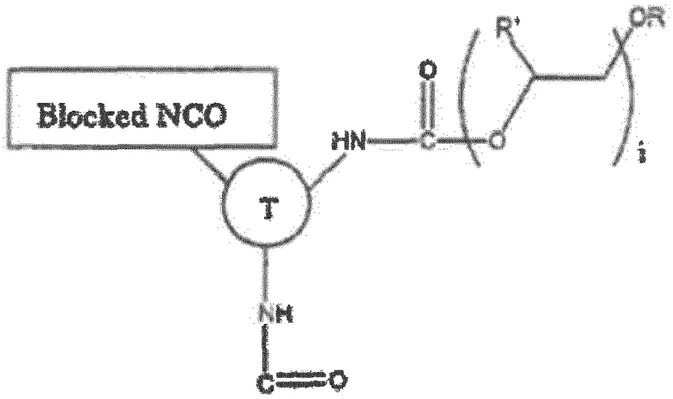

(3)

In structural formulae (1) to (3), R represents an alkyl group, R' represents a hydrogen atom or a methyl group, Blocked NCO represents a group in which an isocyanate group is blocked by a blocking agent, and T represents an isocyanate type or adduct type triisocyanate skeleton. n = 1-5, a, b, c, d = 0-4, e, f, g, h = 0-4, a + e, b + f, c + g, d + h = 1-4, i = 4 - 50, wherein the ratio of the building blocks (1) to (3) may vary. In preferred embodiments, the blocked isocyanate comprises the terminating group A according to structural formula (1) in a range of 59 to 88%, the building block according to structural formula (2) in a range of 2 to 14%, and the building block according to structural formula (3) in a range of 10 to 30%, wherein the sum of terminating group A according to structural formula (1) and the building block according to structural formula (2) is in the range of 70-90% based on the total molecular weight of the blocked isocyanate. In another more preferred embodiment of the present invention, the triisocyanate compound is a trimethylolpropane adduct of tolylene diisocyanate.

In a preferred embodiment of the invention, the textile material comprises cationic blocked isocyanate, preferably Meikanate, in a relative amount per weight based on weight of fabric of at least 1 wt.-%, preferably of at least 2 wt.-%, more preferably of at least 4 wt.-%, even more preferably of at least 6 wt.-%, most preferably of at least 8 wt.-% and/or of at most 30 wt.-%, preferably of at most 20 wt.-%, more preferably of at most 15 wt.-%, even more preferably of at most 12 wt.-%, most preferably of at most 8 wt.-%.

Other crosslinkers may be, for example acrylic or polyurethane binders which are hydrophobic in nature. Acrylic crosslinkers may, for example be styrene acrylic ester copolymers provided in aqueous dispersion. Polyurethane (PU) binders may, for example, be aqueous polyester polyurethane dispersions

***Further components comprised by the textile material***

In preferred embodiments of the invention, the textile material comprises at least one acid selected from the group consisting of acetic acid, citric acid, salicylic acid, benzoic acid and sorbic acid, or salts thereof, in particular sodium salts thereof, preferably citric or acetic acid, most preferably citric acid. Without wishing to be bound by theory, the acid provides for affinity of the textile surface so that agents can better adhere to it. For example, the acid may help attacking the fiber material to adhere other agents to it. Also, acid itself may have an antimicrobial effect. Thus, both antimicrobial

efficiency and/or reduction or prevention of odor formation from urine can be enhanced.

In a preferred embodiment of the invention, the textile material comprises acid, preferably citric acid, in a relative amount per weight based on weight of fabric of at least 0.01 wt.-%, preferably of at least 0.03 wt.-%, more preferably of at least 0.06 wt.-%, even more preferably of at least 0.08 wt.-%, most preferably of at least 0.12 wt.-% and/or of at most 1 wt.-%, preferably of at most 0.5 wt.-%, more preferably of at most 0.2 wt.-%, even more preferably of at most 0.15 wt.-%, most preferably of at most 0.12 wt.-%.

A hydrophilic agent may advantageously be adhered to the textile. In preferred embodiments, the hydrophilic agent agent increases the horizontal wicking rate of the textile material by at least 10%, in more preferred embodiments by at least 20%, in even more preferred embodiments by at least 30%, particularly by at least 50%, and in most preferred embodiments by at least 100%, when compared to the same textile material not comprising the hydrophilic agent. The wicking rate in preferred embodiments is measured according to AATCC test method 198-2013. Furthermore, in preferred embodiments, the hydrophilic agent agent decreases the absorbency time of the textile material by at least 20%, in more preferred embodiments by at least 40%, in even more preferred embodiments by at least 60%, and most preferred embodiments by at least 80%, when compared to the same textile material not comprising the hydrophilic agent. The absorbency time in preferred embodiments is measured according to AATCC test method 79-2014 (Option A). The hydrophilic agent is may be polyethylene glycol (PEG) selected from PEG-400, PEG-500, PEG-600 and/or PEG-800, most preferably PEG-400. This allows for a liquid, in particular a body fluid such as blood and/or urine to be more quickly and more evenly distributed in the textile material of the invention. Also, the hydrophilic agent allows maintaining the hydrophilicity, in particluar the wicking rate, which may be reduced by the presence of hydrophobic agents such as crosslinkers or binders.

For example, the textile material of the present invention may comprise a hydrophilic agent, e.g. polyethylene glycol (PEG) selected from PEG-400, PEG-500, PEG-600 and/or PEG-800, preferably PEG-400, in a relative amount per weight based on weight of fabric of at least 2 wt.-%, preferably of at least 4 wt.-%, more preferably of at least 8 wt.-%, even more preferably of at least 12 wt.-%, most preferably of at least 16 wt.-% and/or of at most 60 wt.-%, preferably of at most 40 wt.-%, more preferably of at most 30 wt.-%, even more preferably of at most 24 wt.-%, most preferably of at most 16 wt.-%.

The textile material may advantageously comprise a fabric softener, preferably a silicone, more preferably a functionalized silicone, even more preferably an amino-functionalized silicone, most preferably Ultratex®.

***Preferred types of textile materials and fabrics***

The textile material may be a fiber, a yarn, or a fabric, preferably a fabric, for example a woven, knitted, warp-knitted, or crocheted fabric. In preferred embodiments of the invention, the textile material is a non-woven fabric, such as a bonded or spun bonded or melt blown or hot melted fabric, and any combination thereof.

The textile material may be a cellulosic material, a synthetic material or a blend of cellulosic and synthetic material. Preferable is a synthetic material. Cellulosic material may comprise cotton, viscose, rayon, linen, hemp, ramie, jute, or combinations (blends) thereof, preferable is viscose. The synthetic material may comprise polyester, polyamide (nylon), acrylic polyester, spandex (elastane, Lycra), aramids, modal, sulfar, polylactide (PLA), lyocell, polybutyl tetrachloride (PBT), polypropylene (PP), or combinations (blends) thereof. Preferable is polypropylene (PP).

The present invention may be applied to a wide range of fabrics and textile materials, making it a versatile technology for many different types of applications.

***Method of manufacturing a textile material of the invention***

The textile material of the present invention may be manufactured by treating the starting textile material using a liquor application process, preferably a padding process, which is inexpensive.

The treated textile material is then typically heat-treated at a temperature of at least 80 °C, preferably at least 100 °C, more preferably at least 110 °C, and most preferably at least about 120 °C and/or at a heat-treatment temperature of at most 160 °C, preferably at most 140 °C, more preferably at most 130 °C, and most preferably at most about 120 °C. Heat-treatment is preferably conducted for a time period of at least 60 seconds, preferably at least 90 seconds, more preferably at least 120 seconds, even more preferably at least 180 seconds, and most preferably for at least 240 seconds and/or for a heat treatment time of at most 240 seconds, preferably at most 200 seconds, more preferably at most 180 seconds, even more preferably at most 150 seconds, and most preferably at most 120 seconds. This provides for efficient adherence of the one or more agents to the textile material and/or crosslinking of the one or more agents and/or the one or more crosslinkers.

The liquor of the liquor application process comprises one or more or all of the components as defined for the textile material of any one of the embodiments described above. The inventors found that with the following concentrations, the agents can be applied to the textile material in sufficient amounts for providing the desired effects, namely an

antimicrobial effect and/or reduction and/or prevention of odors from urine:
In the preferred embodiments of the present invention, the liquor of the liquor application process comprises polyDADMAC in an amount of at least 5 $g \cdot L^{-1}$, preferably at least 10 $g \cdot L^{-1}$, more preferably at least 20 $g \cdot L^{-1}$, even more preferably at least 30 $g \cdot L^{-1}$, and most preferably at least 40 $g \cdot L^{-1}$ and/or in an amount of at most 1000 $g \cdot L^{-1}$, preferably at most 300 $g \cdot L^{-1}$, more preferably at most 200 $g \cdot L^{-1}$, even more preferably at most 150 $g \cdot L^{-1}$, and most preferably at most 100 $g \cdot L^{-1}$.

In another embodiment of the present invention, the liquor of the liquor application process comprises chitosan in an amount of at least 1 $g \cdot L^{-1}$, preferably at least 5 $g \cdot L^{-1}$, more preferably at least 10 $g \cdot L^{-1}$, even more preferably at least 15 $g \cdot L^{-1}$, and most preferably at least 20 $g \cdot L^{-1}$ and/or in an amount of at most 100 $g \cdot L^{-1}$, preferably at most 60 $g \cdot L^{-1}$, more preferably at most 40 $g \cdot L^{-1}$, even more preferably at most 30 $g \cdot L^{-1}$, and most preferably at most 20 $g \cdot L^{-1}$.

In the preferred embodiments of the present invention, the liquor of the liquor application process comprises Meikanate in an amount of at least 1 $g \cdot L^{-1}$, preferably at least 5 $g \cdot L^{-1}$, more preferably at least 10 $g \cdot L^{-1}$, even more preferably at least 15 $g \cdot L^{-1}$, and most preferably at least 20 $g \cdot L^{-1}$ and/or in an amount of at most 100 $g \cdot L^{-1}$, preferably at most 60 $g \cdot L^{-1}$, more preferably at most 40 $g \cdot L^{-1}$, even more preferably at most 30 $g \cdot L^{-1}$, and most preferably at most 20 $g \cdot L^{-1}$.

Also, in the preferred embodiments of the present invention, the liquor of the liquor application process comprises citric acid or acetic acid in an amount of at least 0.05 $g \cdot L^{-1}$, preferably at least 0.1 $g \cdot L^{-1}$, more preferably at least 0.15 $g \cdot L^{-1}$, even more preferably at least 0.2 $g \cdot L^{-1}$, and most preferably at least 0.3 $g \cdot L^{-1}$ and/or in an amount of at most 1 $g \cdot L^{-1}$, preferably at most 0.7 $g \cdot L^{-1}$, more preferably at most 0.5 $g \cdot L^{-1}$, even more preferably at most 0.4 $g \cdot L^{-1}$, and most preferably at most 0.3 $g \cdot L^{-1}$.

The liquor of the liquor application process may also comprises PEG-400 in an amount of at least 10 $g \cdot L^{-1}$, preferably at least 20 $g \cdot L^{-1}$, more preferably at least 30 $g \cdot L^{-1}$, even more preferably at least 40 $g \cdot L^{-1}$, and most preferably at least 50 $g \cdot L^{-1}$ and/or in an amount of at most 200 $g \cdot L^{-1}$, preferably at most 150 $g \cdot L^{-1}$, more preferably at most 100 $g \cdot L^{-1}$, even more preferably at most 75 $g \cdot L^{-1}$, and most preferably at most 50 $g \cdot L^{-1}$.

***Products comprising the textile material*** *of the* ***present invention***

The present invention further relates to products comprising a textile material according to any one of the embodiments described above. In exemplary embodiments, the textile material according to the present invention is comprised in hygiene products or personal care products such as diapers, adult incontinence products, bed pads, underwear liners, or sanitary napkins.

As can be seen in **Fig. 1,** such a hygiene product comprises a first layer **1** comprising a textile material according to the present invention, and preferably a second layer **2** comprising a textile material according to the present invention. During ordinary use of the hygiene product, the first layer **1** is closer to the body of the user than the second layer **2.** The first layer **1** and/or the second layer **2** may be treated polypropylene fabrics.

Preferred embodiments of the hygiene product further comprise a layer of absorber material **3.** During ordinary use of the hygiene product, the first layer **1** and where existent the second layer **2** are closer to the body of the user than the layer of absorber material **3.** The absorber material can be any textile material; preferably the absorber material is or comprises one or more of superabsorbent polymers (SAPs) as known in the art. In this case, layer **3** preferably does not comprise a textile material according to the present invention. However, the layer of absorber material **3** is preferably in direct contact with the first layer **1** or the second layer **2.** Thus, even if bacteria are formed in a layer of absorber material **3** where the textile material of the invention is not present, the above-mentioned enzyme which causes hydrolysis of urea to ammonia and carbon dioxide may be released but can still be inhibited by the presence of a textile material of the invention.

Furthermore, the hygiene product will typically comprise a water-impermeable layer **4.** In this case, the hygiene product is preferably configured such that during its ordinary use, the first layer **1** and where existent the second layer **2** and layer of absorber material **3** are closer to the body of the user than the water-impermeable layer **4.** The water-impermeable layer may comprise any water-impermeable material, preferably a polyethylene (PE) film.

Layers **1** and **2** provide for both an antimicrobial effect and reduction of malodors from urine. The purpose of layer **3** is to absorb a user's body-fluids such as urine and/or blood. Layer **4** prevents undesired leakage from the hygiene product.

***Use of a textile material of the present invention***

A textile material according to the invention may be used for killing or reducing a number of microbes, by bringing the microbes and/or a liquid comprising the microbes into contact with the textile. The liquid may be a body fluid, such as urine or blood. The microbes may be bacteria, virus, or fungi, preferably bacteria. The textile material will typically be comprised by a hygiene product as described above, and the hygiene product will typically be worn by a human user.

A textile material according to the invention may be also be used for reducing malodors of urin. This is achieved by bringing the urine in contact with the textile material, preferably by passing urine through a layer comprising the textile material. Again, the textile material will typically be comprised by a hygiene product as described above, and the hygiene product will typically be worn by a human user.

This way both superior antimicrobial efficiency as well as reduction and/or prevention of odors from urine can be achieved with a textile material according to the invention.

**EXAMPLES**

A series of different textile materials according to the present invention was produced under laboratory conditions closely simulating the method for producing the textile material described hereinabove. The so obtained textile materials were tested for their efficiency of reducing malodor from urine, reducing urea composition, and their antimicrobial efficiency, according to the test protocols stated herein.

***Chemicals used***

Chemicals used in the examples hereinafter comprise the following:
*"polyDADMAC"* refers to Polydamac 40% from Atish Chemicals, India: an aqueous solution containing 40% of polyDADMAC.

*"Chitosan"* from Kraeber & Co GmbH, Germany: pure polyglucosamine (chitosan) used as an aqueous solution containing 5% chitosan in 10% citric acid.

*"Meikanate"* refers to Meikanate CX from Meisei Chemical Works Ltd, Japan: an 70-90% aqueous emulsion of a positively charged blocked isocyanate.

*"Citric acid"* from Jungbunzlaur, Switzerland: used either as anhydrous powder or as 10% aqueous stock solution.

*"UltratexFMW"* from Huntsman: amino-functionalized silicone microemulsions used as received.

*"PEG-400"* refers to Polyethylene glycol having an average molecular weight of 400 g/mol from Merck.

"Acrylic binder" refers to Appretan NTR6553 liq from Archroma: an aqueous styrene acrylic copolymer dispersion.

"Polyurethane binder" refers to Lurapret N-DPS liq from Archcroma: an aqueous polyester polyurethane dispersion.

"Arginine" refers to L-arginine from Thermo Fisher Scientific used as received.

Specific examples for preparing textile materials according to the present invention are given below. Quantities of components are given either as relative amounts, i.e. in % (meaning wt.-% based on the amount of water in case of aqueous solutions), or the expression "gpl", "g/L" of "g-$L^{-1}$" (grams per liter). The expression "gpl" or "g/L" "g-$L^{-1}$" (grams per liter) refers to the weight of the respective chemicals as specified above in 1 liter of the preparation. The final concentration of each antimicrobial active ingredient can be calculated from the quantities of the respective components. E.g., "100 gpl polyDADMAC" refers to 100 g/L of Polydadmac 40%, which, as mentioned above, comprises 40% polyDADMAC. This means that 40% x 100 g/L = 40 g/L (40% x 100 gpl = 40 gpl) of polyDADMAC were used.

***Protocol for testing the antimicrobial efficacy***

The underlying test protocol is publicly available from the American Association of Textile Chemists and Colorist (AATCC). Specifically, tests according to AATCC 100-2012 (Assessment of Antimicrobial Finishes on Textile Materials) were performed. All microbiological tests were performed with cultures of the bacteria *Escherichia coli* American Type Culture Collection (ATCC) 25922 as well as with cultures of the bacteria *Staphylococcus aureus* ATCC 6538, wherein an 18 hours-old inoculum (2.03 x 1010 CFU/ml; CFU = colony forming units) was used. As neutralizer, a Dey-Engley neutralizing broth was used, and as diluent, a sterile phosphate buffered saline containing 0.5% BSA was used. The samples were sterilized for the tests by autoclaving.

For determination of antimicrobial efficacy, the log reduction of bacteria was calculated from control and test samples according to Formula 1:

$$\text{Log reduction of bacteria} = B - A \qquad (1)$$

wherein,

A = log10 of the number of bacteria recovered from the inoculated samples (5 minutes contact time), and

B = log10 of the number of bacteria recovered from the inoculated samples (0 minutes contact time).

*Protocol for testing the effect on urea decomposition by urease*

The effect on urea decomposition by urease was tested indirectly via conductivity measurement as described in the literature (cf., for example, Ray, D. Staetta, T.H. Boyer, Environ. Sci.: Water Res Technol. 2018, 4, 87). For the experiments, 10 g urea, 5 g shredded fabric (treated polypropylene diaper fabric vs. untreated polypropylene diaper fabric), 0.533 g urease, phosphate buffer (16 mL) and aqueous HCl solution (0.1N) are dissolved in 1 liter of deionized water in a beaker. The slurry is homogenized using a magnetic stirrer set at 350-400 rpm. The temperature is kept at 22-25 °C. The conductivity is measured every 15 minutes. It is noted, that this experimental protocol represents only a close simulation of practical conditions, i.e. of the treated fabric as a layer in a diaper worn by a user. Also, a urea solution is not the same as natural urine. Still, this test provides an indication for the inhibition of urea decomposition by urease.

*Protocol for testing the reduction of malodors from urine*

Baby diapers equipped with one or more layers of a textile material according to the present invention were tested for odor control properties. The top two non-woven layers of commercially available diapers (Pampers, medium size for 7-12 kg) were removed and replaced by two layers of a textile material according to the present invention. In this manner, diapers with the preferred layer structure as described above were created, comprising from top to bottom two layers of a textile material according to the present invention, one layer of superabsorbent polymers, and one water-impermeable layer comprising a PE film. The odor control tests with diapers were carried out according to the following procedure (all examples carried out in duplicate):

1. 80-100 ml of natural urine was poured on each diaper.
2. Diapers were kept inside a clean sterile beaker at 30 °C, covered with aluminum foil and kept for incubation.
3. Every two hours sensual odor observations were recorded.

**WORKED EXAMPLES**

The following tables contain (1) recipes of treatment compositions for treating textile materials and (2) test data regarding antimicrobial efficiency, prevention and/or reduction of odor formation from urine and/or data from conductivity measurements for indirect measurement of reduced urea decomposition.

For the test series, 100%-polypropylene (PP) non-woven fabrics (GSM: 22 g/m$^2$; fiber thickness: 0.15 mm) were used. In a further test series, 100%-polyethylene (PE) non-woven fabrics (GSM: 22 g/m$^2$) and perforated polyethylene films were used.

The fabrics were treated with the different treatment compositions as stated in the tables by a padding process (all amounts of ingredients of the recipes are given in gpl), wherein one nip and dip were carried out at a padding temperature corresponding to room temperature and a padding pressure of 2 bar.

Subsequently, the textile material was subjected to heat-treatment comprising drying for a drying time of 2 minutes at a drying temperature of 120 °C, and cured for a curing time of 2 minutes at a curing temperature of 120 °C. Subsequent to treatment, the textile material samples were subjected to different tests as described below.

Antimicrobial efficiency is given as log kill values as measured according to the AATCC 100 test method for E.coli.

The odor control tests were carried out according to the procedure described above. The odor formation was evaluated according to sensual odor observations (- = no smell, + = slight smell, ++ = moderate smell, +++ = strong smell).

Urease inhibition/urea decomposition was tested by conductivity measurements as described above.

*Treatment compositions comprising one agent*

**Table 1a:**

| Recipe | 1a | 1b | 1c | 1d | C |
|---:|---|---|---|---|---|
| **Component** | | | | | |
| polyDADMAC | 20 | 70 | 100 | | |
| Meikanate | | | | 10 | |

(continued)

| Recipe | 1a | 1b | 1c | 1d | C |
|---|---|---|---|---|---|
| Brooksol XC 870 | | | | | |
| Acrylic binder | | | | | |
| Polyurethane binder | | | | | |
| Chitosan | | | | | |
| BKC | | | | | |
| Arginine | | | | | |
| Citric acid | | | | | |
| Acetic acid | | | | | |
| PEG 400 | | | | | |
| Ultratex FMW | | | | | |
| | | | | | |
| **Fabric type** | PP | PP | PP | PP | PP |
| | | | | | |
| **Antimicrobial test (AATCC 100)** | | | | | |
| 1 h (E.coli) | 3.81 | 2.79 | 2.64 | 0.22 | -0.17 |
| 24 h (E.coli) | 4.56 | 5.34 | >6.12 | -0.34 | -1.03 |
| | | | | | |
| **Odor Test** | | | | | |
| 0 h | "+" | "+" | "+" | "+" | "++" |
| 4 h | "++" | "++" | "++" | "++" | "++" |
| 6 h | "+++" | "++" | "++" | "+++" | "+++" |
| 8 h | "+++" | "+++" | "+++" | "+++" | "+++" |
| 24 h | "+++" | "+++" | "+++" | "+++" | "+++" |
| | | | | | |
| **Urea decomposition** | | | | | |
| Conductivity (µS) $t_i$ = 0 min | 539 | 456 | 323 | 534 | 234 |
| Conductivity (µS) $t_f$ = 285 min | 1289 | 1258 | 1280 | 1264 | 2650 |
| Relative increase | 239 | 276 | 396 | 237 | 1132 |
| Relative reduction | 79 | 76 | 65 | 79 | 0 |

**Table 1b:**

| Recipe | 1e | 1f | 1g | 1h | C |
|---|---|---|---|---|---|
| **Component** | | | | | |
| polyDADMAC | | | | | |
| Meikanate | 20 | | | | |
| Brooksol XC 870 | | | | | |
| Acrylic binder | | | | | |

(continued)

| Recipe | 1e | 1f | 1g | 1h | C |
|---|---|---|---|---|---|
| **Component** | | | | | |
| Polyurethane binder | | | | | |
| Chitosan | | | | | |
| BKC | | | | | |
| Arginine | | | | | |
| Citric acid | | 0.3 | 1 | | |
| Acetic acid | | | | | |
| PEG 400 | | | | 50 | |
| Ultratex FMW | | | | | |
| | | | | | |
| **Fabric type** | PP | PP | PP | PP | PP |
| | | | | | |
| **Antimicrobial test (AATCC 100)** | | | | | |
| 1 h (E.coli) | 0.24 | 0.31 | 0.13 | 0.41 | -0.17 |
| 24 h (E.coli) | -0.21 | 0.39 | -0.25 | 0.49 | -1.03 |
| | | | | | |
| **Odor Test** | | | | | |
| o h | "+" | "+" | "+" | "+" | "++" |
| 4 h | "++" | "++" | "++" | "++" | "++" |
| 6h | "+++" | "+++" | "+++" | "+++" | "+++" |
| 8 h | "+++" | "+++" | "+++" | "+++" | "+++" |
| 24 h | "+++" | "+++" | "+++" | "+++" | "+++" |
| | | | | | |
| **Urea decomposition** | | | | | |
| Conductivity ($\mu$S) $t_i$ = 0 min | 521 | 556 | 556 | 555 | 234 |
| Conductivity ($\mu$S) $t_f$ = 285 min | 1264 | 1201 | 1201 | 1264 | 2650 |
| Relative increase | 243 | 216 | 216 | 228 | 1132 |
| Relative reduction | 79 | 81 | 81 | 80 | 0 |

The test data provided in Tables 1a and 1b show that treatment compositions with only one agent already provide for a moderate to good relative reduction of urea decomposition compared to untreated textile material, when measured indirectly via conductivity measurement. More importantly, treatment compositions with only polyDADMAC unexpectedly provide for good to excellent results regarding the antimicrobial efficiency.

***Treatment compositions comprising two agents***

Table 2a:

| Recipe | 2a | 2b | 2c | 2d | 2e | C |
|---|---|---|---|---|---|---|
| **Component** | | | | | | |
| polyDADMAC | 100 | 100 | 100 | 100 | 100 | |
| Meikanate | 20 | | | | | |
| Brooksol XC 870 | | 20 | | | | |
| Acrylic binder | | | | | | |
| Polyurethane binder | | | | | | |
| Chitosan | | | | | | |
| BKC | | | | | | |
| Arginine | | | | | | |
| Citric acid | | | 0.3 | 0.3 | 0.3 | |
| Acetic acid | | | | | | |
| PEG 400 | | | | | | |
| Ultratex FMW | | | | | | |
| | | | | | | |
| **Fabric type** | PP | PP | PP | PP | PP | PP |
| | | | | | | |
| **Antimicrobial test (AATCC 100)** | | | | | | |
| 1 h (E.coli) | 2.77 | 2.59 | 2.71 | 2.71 | - | -0.17 |
| 24 h (E.coli) | 3.71 | 3.94 | 3.46 | 3.68 | >5.63 | -1.03 |
| | | | | | | |
| **Odor Test** | | | | | | |
| 0 h | "+" | "+" | "+" | "+" | | "++" |
| 4 h | "+" | "+" | "++" | "+" | | "++" |
| 6 h | "+" | "+" | "++" | "++" | | "+++" |
| 8 h | "+" | "+" | "+++" | "++" | | "+++" |
| 24 h | "+" | "++" | "+++" | "+++" | | "+++" |
| | | | | | | |
| **Urea decomposition** | | | | | | |
| Conductivity ($\mu$S) $t_i$ = 0 min | | | | | 323 | 234 |
| Conductivity ($\mu$S) $t_f$ = 285 min | | | | | 1280 | 2650 |
| Relative increase | | | | | 396 | 1132 |
| Relative reduction | | | | | 65 | 0 |

**Table 2b:**

| Recipe | 2f | 2g | 2h | 2i | C |
|---|---|---|---|---|---|
| **Component** | | | | | |
| polyDADMAC | | | | | |
| Meikanate | 20 | | | | |
| Brooksol XC 870 | | 20 | | | |
| Acrylic binder | | | 20 | | |
| Polyurethane binder | | | | 20 | |
| Chitosan | | | | | |
| BKC | | | | | |
| Arginine | | | | | |
| Citric acid | 0.3 | 0.3 | 0.3 | 0.3 | |
| Acetic acid | | | | | |
| PEG 400 | | | | | |
| Ultratex FMW | | | | | |
| | | | | | |
| **Fabric type** | PP | PP | | | PP |
| | | | | | |
| **Antimicrobial test (AATCC 100)** | | | | | |
| 1 h (E.coli) | 0.31 | 0.43 | 0.21 | -0.03 | -0.17 |
| 24 h (E.coli) | -0.15 | -0.14 | -0.51 | -0.73 | -1.03 |
| | | | | | |
| **Odor Test** | | | | | |
| 0 h | "+" | "+" | "+" | "+" | U/T |
| 4 h | "+" | "+" | "+++" | "++" | "++" |
| 6 h | "++" | "++" | to "+++" | "+++" | "+++" |
| 8 h | "++" | "++" | "+++" | "+++" | "+++" |
| 24 h | "+++" | "+++" | "+++" | "+++" | "+++" |

A comparison of the test data for textile materials treated with compositions comprising only polyDADMAC (recipes 1a to 1c in Table 1a) and a combination of polyDADMAC with Meikanate (recipe 2a in Table 2a) shows that the antimicrobial efficiency of polyDADMAC is slightly reduced in the presence of Meikanate if no other agent is present. However, it is considerd to be still good. On the other hand, the data from tables 2a and 2b show that treatment compositions comprising polyDADMAC and Meikanate surprisingly allow for excellent reduction of odor formation, while other combinations comprising only one (Tables 1a and 1b) or two agents, e.g. polyDADMAC and citric acid, do not.

***Treatment compositions comprising three agents***

*a) Variation of the amount of Meikanate*

**Table 3a:**

| Recipe Component | 3a-1 | 3a-2 | 3a-3 | 3a-4 | 3a-5 | 3a-6 | C |
|---|---|---|---|---|---|---|---|
| polyDADMAC | 100 | 100 | 100 | 100 | 100 | 100 | |
| Meikanate | **15** | **20** | **20** | **20** | **40** | **100** | |
| Brooksol XC 870 | | | | | | | |
| Acrylic binder | | | | | | | |
| Polyurethane binder | | | | | | | |
| Chitosan | | | | | | | |
| BKC | | | | | | | |
| Arginine | | | | | | | |
| Citric acid | 0.3 | 0.3 | 0.3 | 0.3 | 0.3 | 0.3 | |
| Acetic acid | | | | | | | |
| PEG 400 | | | | | | | |
| Ultratex FMW | | | | | | | |
| | | | | | | | |
| **Fabric type** | PP | PP | PP | PP | PP | PP | PP |
| | | | | | | | |
| **Antimicrobial test (AATCC 100)** | | | | | | | |
| 1 h (E.coli) | 3.08 | 2.51 | 2.01 | 2.06 | 1.91 | 1.69 | -0.17 |
| 24 h (E.coli) | >6.11 | 3.87 | >5.63 | >5.95 | >6.11 | >5.95 | -1.03 |
| | | | | | | | |
| **Odor Test** | | | | | | | |
| 0 h | "-" | "+" | | | "-" | | "++" |
| 4 h | "+" | "+" | | | "+" | | "++" |
| 6 h | "+" | "+" | | | "+" | | "+++" |
| 8 h | "+" | "+" | | | "+" | | "+++" |
| 24 h | "++" | "+" | | | "+" | | "+++" |
| **Urea decomposition** | | | | | | | |
| Conductivity (μS) $t_i$ = 0 min | 268 | | 262 | | 261 | | 234 |
| Conductivity (μS) $t_f$ = 285 min | 893 | | 878 | | 792 | | 2650 |
| Relative increase | 333 | | 335 | | 303 | | 1132 |
| Relative reduction | 71 | | 70 | | 73 | | o |

The test data provided in Table 3a show that textile materials treated with compositions comprising polyDADMAC, Meikanate and citric acid surprisingly allow for both excellent antimicrobial efficiency and reduction of odor formation.

*b) Variation of the type and amount of acid*

**Table 3b:**

| Recipe | 3b-1 | 3b-2 | 3b-3 | 3b-4 | 3b-5 | 3b-6 | C |
|---|---|---|---|---|---|---|---|
| **Component** | | | | | | | |
| polyDADMAC | 100 | 100 | 100 | 100 | 100 | 100 | |
| Meikanate | 20 | 20 | 20 | 20 | 20 | 20 | |
| Brooksol XC 870 | | | | | | | |
| Acrylic binder | | | | | | | |
| Polyurethane binder | | | | | | | |
| Chitosan | | | | | | | |
| BKC | | | | | | | |
| Arginine | | | | | | | |
| Citric acid | **0,15** | **0,3** | **0,5** | | | | |
| Acetic acid | | | | **0,15** | **0,3** | **0,5** | |
| PEG 400 | | | | | | | |
| Ultratex FMW | | | | | | | |
| **Fabric type** | PP | PP | PP | PP | I PP | PP | PP |
| | | | | | | | |
| **Antimicrobial test (AATCC 100)** | | | | | | | |
| 1 h (E.coli) | 1.79 | 2.51 | 1.71 | 2.11 | 1.92 | 1.71 | -0.17 |
| 24 h (E.coli) | >6.11 | 3.87 | 4.92 | >6.11 | >6.11 | >6.11 | -1.03 |
| | | | | | | | |
| **Odor Test** | | | | | | | |
| 0 h | "-" | "+" | "-" | "-" | "-" | "-" | "++" |
| 4 h | "+" | "+" | "-" | "+" | "-" | "+" | "++" |
| 6 h | "+" | "+" | "-" | "+" | "-" | "+" | "+++" |
| 8 h | "+" | "+" | "+" | "+" | "+" | "+" | "+++" |
| 24 h | "+" | "+" | "+" | "+" | "+" | "+" | "+++" |
| | | | | | | | |
| **Urea decomposition** | | | | | | | |
| Conductivity (μS) $t_i$ = 0 min | 265 | | 258 | 264 | 261 | 268 | 234 |
| Conductivity (μS) $t_f$ = 285 min | 911 | | 878 | 911 | 897 | 878 | 2650 |
| Relative increase | 344 | | 340 | 345 | 344 | 328 | 1132 |
| Relative reduction | 70 | | 70 | 70 | 70 | 71 | 0 |

The comparison of recipes 3b-1 to 3b-6 in Table 3b shows that both citric acid and acetic acid in combination with polyDADMAC and Meikanate provide for excellent results regarding antimicrobial efficiency and prevention of odors.

*c) Variation of the amount of polyDADMAC*

**Table 3c:**

| Recipe | 3c-1 | 3c-2 | 3c-3 | 3c-4 | 3c-5 | C |
|---|---|---|---|---|---|---|
| **Component** | | | | | | |
| polyDADMAC | **40** | **50** | **100** | **200** | **1000** | |
| Meikanate | 20 | 20 | 20 | 20 | 20 | |
| Brooksol XC 870 | | | | | | |
| Acrylic binder | | | | | | |
| Polyurethane binder | | | | | | |
| Chitosan | | | | | | |
| BKC | | | | | | |
| Arginine | | | | | | |
| Citric acid | 0.3 | 0.3 | 0.3 | 0.3 | 0.3 | |
| Acetic acid | | | | | | |
| PEG 400 | | | | | | |
| Ultratex FMW | | | | | | |
| | | | | | | |
| **Fabric type** | **PP** | PP | PP | PP ' | PP | PP |
| | | | | | | |
| **Antimicrobial test (AATCC 100)** | | | | | | |
| 1 h (E.coli) | 3.73 | 1.41 | 2.51 | 1.71 | >5.74 | -0.17 |
| 24 h (E.coli) | >6.11 | 5.8 | 3.87 | >6.11 | >5.63 | -1.03 |
| | | | | | | |
| **Odor Test** | | | | | | |
| o h | "-" | "-" | "+" | "-" | | "++" |
| 4 h | "+" | "+" | "+" | "+" | | "++" |
| 6 h | "+" | "+" | "+" | "+" | | "+++" |
| 8 h | "+" | "+" | "+" | "+" | | "+++" |
| 24 h | "+" | "+" | "+" | "++" | | "+++" |
| | | | | | | |
| **Urea decomposition** | | | | | | |
| Conductivity (μS) $t_i$ = 0 min | 264 | 258 | | 315 | 769 | 234 |
| Conductivity (μS) $t_f$ = 285min | 991 | 801 | | 1205 | 1740 | 2650 |
| Relative increase | 375 | 310 | | 383 | 226 | 1132 |
| Relative reduction | 67 | 73 | | 66 | 80 | 0 |

*d) Variation of the amount of both polyDADMAC and Meikanate crosslinker*

**Table 3d:**

| Recipe | 3d-1 | 3d-2 | 3d-3 | C |
|---|---|---|---|---|
| **Component** | | | | |
| polyDADMAC | 100 | 50 | 20 | |
| Meikanate | 20 | 10 | 20 | |
| Brooksol XC 870 | | | | |
| Acrylic binder | | | | |
| Polyurethane binder | | | | |
| Chitosan | | | | |
| BKC | | | | |
| Arginine | | | | |
| Citric acid | 0.3 | 0.3 | 0.3 | |
| Acetic acid | | | | |
| PEG 400 | | | | |
| Ultratex FMW | | | | |
| | | | | |
| **Fabric type** | PP | PP | PP | PP |
| | | | | |
| **Antimicrobial test (AATCC 100)** | | | | |
| 1 h (E.coli) | 2.51 | 1.61 | 2.02 | -0.21 |
| 24 h (E.coli) | 3.87 | 2.46 | >5.95 | -1.18 |
| | | | | |
| **Odor Test** | | | | |
| 0 h | "+" | | | "++" |
| 4 h | "+" | | | "++" |
| 6 h | "+" | | | "+++" |
| 8 h | "+" | | | "+++" |
| 24 h | "+" | | | "+++" |
| | | | | |
| **Urea decomposition** | | | | |
| Conductivity (μS) $t_i$ = 0 min | | 148 | | |
| Conductivity (μS) $t_f$ = 285 min | | 584 | | |
| Relative increase | | 395 | | |
| Relative reduction | | 65 | | |

*e) Variation of the type of crosslinker or binder*

**Table 3e:**

| Recipe | 3e-1 | 3e-2 | 3e-3 | C |
|---|---|---|---|---|
| **Component** | | | | |
| polyDADMAC | 100 | 100 | 100 | |
| Meikanate | | | | |
| Brooksol XC 870 | 20 | | | |
| Acrylic binder | | 20 | | |
| Polyurethane binder | | | 20 | |
| Chitosan | | | | |
| BKC | | | | |
| Arginine | | | | |
| Citric acid | 0,3 | 0,3 | 0,3 | |
| Acetic acid | | | | |
| PEG 400 | | | | |
| Ultratex FMW | | | | |
| | | | | |
| **Fabric type** | PP | PP | PP | PP |
| | | | | |
| **Antimicrobial test (AATCC 100)** | | | | |
| 1 h (E.coli) | 2.37 | 1.24 | 0.21 | -0.21 |
| 24 h (E.coli) | 4.92 | 1.41 | -0.61 | -1.18 |
| | | | | |
| **Odor Test** | | | | |
| 0 h | "+" | "+" | "+" | "++" |
| 4 h | "+" | "+" | "+" | "++" |
| 6 h | "+" | "+" | "+" | "+++" |
| 8 h | "+" | "+" | "+" | "+++" |
| 24 h | "+" | "+" | "+" | "+++" |

The comparison of recipes 3e-1 to 3e-3 in Table 3e shows that all indicated combinations of polyDADMAC, citric acid and a crosslinker or binder result in excellent prevention of odors. Remarkably, only the combination of polyDADMAC, citric acid and a blocked isocyanate crosslinker like Meikanate (cf. recipe 3d-1 in Table 3d) or Brooksol XC 870 (recipe 3e-1 in Table 3e) also allow for excellent antimicrobial efficiency in addition to prevention of odors.

*f) Compositions additionally comprising PEG-400*

**Table 3f:**

| Recipe | 3f-1 | 3f-2 | 3f-3 | 3f-4 | 3f-5 | 3f-6 | 3f-7 | C |
|---|---|---|---|---|---|---|---|---|
| **Component** | | | | | | | | |
| polyDADMAC | 100 | 1000 | 100 | 100 | 20 | 100 | 100 | |
| Meikanate | 20 | 20 | 20 | 100 | 20 | | | |
| Brooksol XC 870 | | | | | | | | |
| Acrylic binder | | | | | | 20 | | |
| Polyurethane binder | | | | | | | 20 | |
| Chitosan | | | | | | | | |
| BKC | | | | | | | | |
| Arginine | | | | | | | | |
| Citric acid | 0.3 | 0.3 | 0.15 | 0.3 | 0.3 | 0.3 | 0.3 | |
| Acetic acid | | | | | | | | |
| **PEG 400** | **50** | **50** | **50** | **50** | **50** | **50** | **50** | |
| Ultratex FMW | | | | | | | | |
| | | | | | | | | |
| **Fabric type** | PP | PP | PP | PP | PP | PP | PP | PP |
| | | | | | | | | |
| **Antimicrobial test (AATCC 100)** | | | | | | | | |
| 1 h (E.coli) | 2.53 | 3.24 | 2.62 | 4.19 | 4.15 | 1.13 | 0.12 | -0.17 |
| 24 h (E.coli) | 4.34 | >5.97 | 3.35 | >5.97 | 5.22 | 1.71 | -0.52 | -1.03 |
| | | | | | | | | |
| **Odor Test** | | | | | | | | |
| 0 h | "+" | "+" | "+" | | "+" | "+" | "+" | U/T |
| 4 h | "+" | "++" | "++" | | "++" | "+" | "+" | "++" |
| 6 h | "+" | "+++" | "++" | | "++" | "+" | "+" | "+++" |
| 8 h | "+" | "+++" | "+++" | | "+++" | "+" | "+" | "+++" |
| 24 h | "+" | "+++" | "+++" | | "+++" | "+" | "+" | "+++" |

As can be derived from the data shown in Tables 3a to 3f, PEG-400 does not negatively affect the antimicrobial efficiency of the treated fabrics.

*g) Compositions additionally comprising fabric softener and PEG-400*

**Table 3g:**

| **Recipe** | **3g-1** | **3g-2** | **3g-3** | **3g-4** | **3g-5** | **3g-6** | **C** |
|---|---|---|---|---|---|---|---|
| **Component** | | | | | | | |
| polyDADMAC | 20 | 30 | 50 | 20 | 30 | 50 | |
| Meikanate | 20 | 20 | 20 | 20 | 20 | 20 | |
| Brooksol XC 870 | | | | | | | |
| Acrylic binder | | | | | | | |
| Polyurethane binder | | | | | | | |
| Chitosan | | | | | | | |
| BKC | | | | | | | |
| Arginine | | | | | | | |
| Citric acid | 0.3 | 0.3 | 0.3 | 0.3 | 0.3 | 0.3 | |
| Acetic acid | | | | | | | |
| PEG 400 | | | | 50 | 50 | 50 | |
| Ultratex FMW | 50 | 50 | 50 | 50 | 50 | 50 | |
| | | | | | | | |
| **Fabric type** | PP | PP | PP | PP | PP | PP | PP |
| | | | | | | | |
| **Antimicrobial test (AATCC 100)** | | | | | | | |
| 1 h (E.coli) | 3.55 | 2.84 | 2.26 3.55 | | 2.84 | 2.26 | -0.17 |
| 24 h (E.coli) | 4.29 | 5.07 | 4.97 | 4.29 | 5.07 | 4.97 | -1.03 |
| **Odor Test** | | | | | | | |
| 0 h | | | | "+" | "+" | "+" | U/T |
| 4 h | | | | "++" | "++" | "++" | "++" |
| 6 h | | | | "+++" | "+++" | "+++" | "+++" |
| 8 h | | | | "+++" | "+++" | "+++" | "+++" |
| 24 h | | | | "+++" | "+++" | "+++" | "+++" |
| | | | | | | | |
| **Urea decomposition** | | | | | | | |
| Conductivity ($\mu$S) $t_i$ = 0 min | 245 | 236 | 236 | 269 | 240 | 268 | 234 |
| Conductivity ($\mu$S) $t_f$ = 285 min | 709 (75 min) | 713 (75 min) | 713 (75 min) | 1200 | 1355 | 1330 | 2650 |
| Relative increase | 289 (75 min) | 302 (75 min) | 302 (75 min) | 446 | 565 | 496 | 1132 |
| Relative reduction | 37 (75 min) | 35 (75 min) | 35 (75 min) | 61 | 50 | 56 | 0 |

As can be derived from the data for recipes 3g-1 to 3g-6 presented in Table 3g above, the presence of fabric softener does not negatively affect the antimicrobial efficiency.

*h) Compositions comprising different antimicrobial agents*

**Table 4:**

| Recipe | 4-1 | 4-2 | 4-3 | 4-4 | 4-5 | C |
|---|---|---|---|---|---|---|
| **Component** | | | | | | |
| polyDADMAC | 20 | | | | 20 | I |
| Meikanate | 20 | 20 | 20 | | 20 | |
| Brooksol XC 870 | | | | | | |
| Acrylic binder | | | | | | |
| Polyurethane binder | | | | | | |
| Chitosan | 20 | 50 | 100 | 100 | 20 | |
| BKC | | | | | | |
| Arginine | | 50 | | 20 | | |
| Citric acid | 0.3 | 0.3 | 0.3 | 0.3 | 0.3 | |
| Acetic acid | | | | | | |
| PEG 400 | | | | | 50 | |
| Ultratex FMW | | | | | | |
| | | | | | | |
| **Fabric type** | PP | PP | PP | PP | PP | PP |
| | | | | | | |
| **Antimicrobial test (AATCC 100)** | | | | | | |
| 1 h (E.coli) | 1.78 | 1.47 | 5.72 | 1.64 | 3.04 | -0.17 |
| 24 h (E.coli) | 3.44 | 3.55 | >6.02 | 4.84 | 3.46 | -1.03 |
| | | | | | | |
| **Odor Test** | | | | | | |
| o h | | "++" | "+" | "+" | | "++" |
| 4 h | | "++" | "+" | "+" | | "++" |
| 6 h | | "+++" | "++" | "+" | | "+++" |
| 8 h | | "+++" | "++" | "++" | | "+++" |
| 24 h | | "+++" | "++" | "++" | | "+++" |

As can be derived from the data for recipes 4-1 to 4-5 presented in Table 4 above, all antimicrobial agents used allow for good to excellent antimicrobial efficiency. However, odor prevention is provided only to some extent with recipes 4-3 and 4-4. In comparison, the combination of polyDADMAC, citric acid and Meikanate is superior as it provides for both excellent antimicrobial efficiency and odor prevention (cf. recipe 3d-1 in Table 3d).

*i) Treatment of polyethylene (PE)fabrics*

Table 5:

| Recipe | 5-1 | 5-2 | 5-3 | 5-4 | C | C |
|---|---|---|---|---|---|---|
| **Component** | | | | | | |
| polyDADMAC | 10 | 10 | 10 | 10 | | |
| Meikanate | 20 | 20 | | | | |
| Brooksol XC 870 | | | | | | |
| Acrylic binder | | | | | | |
| Polyurethane binder | | | | | | |
| Chitosan | 20 | | | | | |
| BKC | 10 | 10 | 10 | 10 | | |
| Arginine | | | | | | |
| Citric acid | 0.3 | 0.3 | 0.3 | 0.3 | | |
| Acetic acid | | | | | | |
| PEG 400 | 20 | | 20 | | | |
| Ultratex FMW | | | | | | |
| | | | | | | |
| **Fabric type** | PP | PE | PE | PE | PE | PE |
| | | | | | | |
| **Antimicrobial test (AATCC 100) E.coli** | | | | | | |
| 10 minutes | 3.39 | 2.86 | 2.13 | 2.63 | -0.05 | -0.07 |
| 4 hr | >4.26 | >4.26 | >4.26 | >4.26 | -0.49 | -0.38 |
| 8 hr | >4.91 | >4.91 | 4.22 | 4.83 | -0.65 | -0.51 |
| 24 hr | >5.14 | >5.14 | >5.14 | 4,45 | -1,76 | -1.5 |
| | | | | | | |
| **Antimicrobial test (AATCC 100) S.aureus** | | | | | | |
| 10 minutes | 3.27 | 2.89 | 2.52 | 2.21 | -0.05 | -0.02 |
| 4 hr | 5.49 | 4.59 | 4.54 | 5.49 | -0.4 | -0.23 |
| 8 hr | >5.49 | >5.49 | >5-49 | >5-49 | -0.51 | -0.35 |
| 24 hr | >5.49 | >5.49 | >5.49 | >5.49 | -0.18 | -0.15 |

The experimental results provided in Table 5 show, that recipes 5-1 to 5-4 applied to polyethylene fabrics provide for excellent antimicrobial efficiency against both E.coli and S.aureus already after 10 minutes. Also, the presence of PEG-400 does not negatively affect the antimicrobial performance (cf. recipes 5-1 vs. 5-2 and 5-3 vs 5-4).

***Tests for estimating pick-up and final amounts of agents on the treated textile material***

For estimating pick-up and final amounts of agents on the treated textile material, textile materials were weighted before and after treatment according to the present invention. The final amount of an agent on the treated textile material is estimated based on the weight difference through treatment and the relative amounts of the chemical substances in the treatment composition, also taking into account the weight difference of an untreated control sample.

The pick-up in wt.-% based on dry weight of textile material is estimated according to the following formula:

$$Pick - up\ (component) = \frac{(\Delta_{sample} - \Delta_{control}) * Rel.\ amount\ in\ treatment\ composition\ (component)}{initial\ weight\ before\ treatment}$$

Table 7 shows the absolute and relative amounts of agents in exemplary treatment compositions as well as weight of textile samples before and after treatment and final amounts of agents of the treated textile materials in wt.-% based on dry weight of textile material ("relative pick-up").

**Table 7:**

| **Recipe** | 7a | | 3c-3 | | 7b | | | |
|---|---|---|---|---|---|---|---|---|
| **Components** | **Amounts used for preparing treatment composition / gpl** | | | | | | | |
| polyDADMAC | 20 | 20 | 100 | 100 | 20 | 20 | | |
| Meikanate | 20 | 20 | 20 | 20 | 20 | 20 | | |
| Chitosan | 0 | 0 | 0 | 0 | 20 | 20 | | |
| Citric acid | 0.3 | 0.3 | 0.3 | 0.3 | 0.3 | 0.3 | | |
| | | | | | | | | |
| **Recipe** | 7a | | 3c-3 | | 7b | | | |
| **Components** | **Absolute amounts of chemical substances in composition** | | | | | | | |
| polyDADMAC | 8.0 | 8.0 | 40.0 | 40.0 | 8.0 | 8.0 | | |
| Meikanate | 18.0 | 18.0 | 18.0 | 18.0 | 18.0 | 18.0 | | |
| Chitosan | 0.0 | 0.0 | 0.0 | 0.0 | 1.0 | 1.0 | | |
| Citric acid | 0.03 | 0.03 | 0.03 | 0.03 | 0.03 | 0.03 | | |
| | | | | | | | | |
| | **Relative amount of chemical substances in composition (solvent not included)** | | | | | | | |
| Components | 7a | | 3c-3 | | 7b | | Control | |
| polyDADMAC | 30.7 | 30.7 | 68.9 | 68.9 | 29.6 | 29.6 | | |
| Meikanate | 69.2 | 69.2 | 31.0 | 31.0 | 66.6 | 66.6 | | |
| Chitosan | 0.0 | 0.0 | 0.0 | 0.0 | 3.7 | 3.7 | | |
| Citric acid | 0.1 | 0.1 | 0.1 | 0.1 | 0.1 | 0.1 | | |
| | | | | | | | | |
| | **Sample weight before and after treatment** | | | | | | | |
| initial weight / g | 0.8 | 0.8 | 0.8 | 0.9 | 0.8 | 0.8 | 0.8 | 0.8 |
| After dry Weight/g | 1.0 | 1.0 | 1.0 | 1.0 | 1.0 | 1.0 | 0.9 | 0.9 |
| Weight difference/g | 0.2 | 0.2 | 0.1 | 0.2 | 0.1 | 0.1 | 0.1 | 0.0 |
| | | | | | | | | |
| | Pick-up /wt.-% | | | | | | | |
| Component | 7a | | 3C-3 | | 7b | | Control | |
| polyDADMAC | 4.9 | 3.9 | 7.7 | 9.2 | 3.0 | 3.0 | 0.0 | 0.0 |
| Meikanate | 11.0 | 8.7 | 3.4 | 4.2 | 6.8 | 6.8 | 0.0 | 0.0 |
| Krabber | 0.0 | 0.0 | 0.0 | 0.0 | 0.4 | 0.4 | 0.0 | 0.0 |
| Citric acid | 0.0 | 0.0 | 0.0 | 0.0 | 0.0 | 0.0 | 0.0 | 0.0 |

### *Effect on urea decomposition by urease*

According to the results of the conductivity measurements shown in Tables 1 to 5 above, in presence of treated fabric, urease activity is inhibited up to 81% compared to a control sample with untreated fabric. The exact mechanism of urea inhibition is still unclear, but it can be caused by adsorption or ionic interaction of the enzyme with the positively charged fabric surface.

### *Odor control tests*

The odor control tests with diapers were carried out according to the procedure described above with recipes 2e, 3a-3c, 3c-5 and 3d-2 shown in Tables 2a, 3a, 3c and 3d. The results of the odor control tests are summarized in below Table 8:

**Table 8:**

| Recipe | | Sample taken after | | | | |
|---|---|---|---|---|---|---|
| Layer 1 | Layer 2 | 0 h | 4 h | 6 h | 8 h | 24 h |
| --- | --- | + | +++ | +++ | +++ | +++ |
| 3a-3 | 2e | + | ++ | ++ | +++ | +++ |
| 3a-3 | 3a-3 | - | - | - | - | + |
| 3c-5 | 3c-5 | - | + | ++ | +++ | +++ |
| 3a-3 | 3d-2 | + | ++ | ++ | +++ | +++ |

Diapers having two layers of fabrics treated with recipes 3a-3 / 2e, 3c-5 /3c-5 or 3a-3 / 3d-2 showed moderate smell at 4 and 6 hours and strong smell at 8 and 24. The diaper with layers of untreated fabrics already showed a strong smell after 4 hours. In contrast, diapers having two layers of non-woven polypropylene fabric treated with recipe 3a-3 surprisingly showed no smell at o, 4, 6 and 8 hours and only very slight smell at 24 hours of incubation.

### *Conclusions from test results*

The data provided in Tables 1a to 5 show that polyDADMAC alone shows an antimicrobial effect but prevents malodors only to a limited extent, while Meikanate alone is neither antimicrobial nor preventing malodors, and citric acid alone is antimicrobial and preventing malodors only to limited extent. Compositions comprising polyDADMAC and a crosslinker or binder (recipes 2a and 2b in Table 2a) provide for good antimicrobial efficiency and excellent reduction of odor formation, while compositions comprising a crosslinker or binder and citric acid and no polyDADMAC do not provide for an antimicrobial effect or prevention of odor formation (cf. recipes 2f to 2i in Table 2b)

**Table 9a:** (- = no effect, (+) = weak effect, + = strong effect)

| Effect | Crosslinker or binder | Citric acid | Combination |
|---|---|---|---|
| Antimicrobial | - | (+) | - |
| Odor prevention | - | (+) | - |

**Table 9b:** (- = no effect, (+) = weak effect, + = strong effect)

| Effect | polyDADMAC | Crosslinker or binder | Combination |
|---|---|---|---|
| Antimicrobial | + | - | (+) |
| Odor prevention | (+) | - | + |

In contrast, the combination of polyDADMAC, Meikanate and citric acid or acetic acid unexpectedly provides for both an antimicrobial effect and efficient prevention of malodors:

**Table 9c:** (- = no effect. (+) = weak effect. + = strong effect)

| Effect | PDM | Meikanate | Citric acid | Combination |
|---|---|---|---|---|
| Antimicrobial | + | - | (+) | + |
| Odor prevention | (+) | - | (+) | + |

As can be derived from the data shown in Tables 1 to 4, polypropylene fabrics treated with polyDADMAC alone or in combination with Meikanate and citric acid or acetic acid allow for excellent log kill values after already 1 hour of up to 3.81 and after 24 hours even up to > 6.12. Also, the combinations of polyDADMAC, Meikanate and citric acid or acetic acid allow for excellent inhibition of malodors, while the single components do not allow for prevention of malodors. Thus, the combination of polyDADMAC, Meikanate and citric acid or acetic acid provides for synergistic inhibition of malodors from urine.

## Claims

**1.** A hygiene product comprising a first layer (1) and preferably a second layer (2) of a textile material to which one or more antimicrobial agents and one or more crosslinkers or binders are adhered.

**2.** The hygiene product of the preceding claim, wherein the one or more antimicrobial agents and one or more crosslinkers or binders prevent or inhibit, when the textile material comes into contact with urine, decomposition of urea, comprised by the urine, by urease.

**3.** The hygiene product of any one of the preceding claims, wherein one or more or preferably all of the crosslinkers or binders and/or antimicrobial agents are not hydrophobic, preferably hydrophilic.

**4.** The hygiene product of any one of the preceding claims, wherein one or more or preferably all of the crosslinkers or binders and/or antimicrobial agents are cationic or non-ionic, preferably cationic.

**5.** The hygiene product of any one of the preceding claims, wherein the one or more crosslinkers or binders are an isocyanate, preferably a blocked isocyanate, more preferably a cationic blocked isocyanate, most preferably Meikanate.

**6.** The hygiene product of any one of the preceding claims, wherein the one or more antimicrobial agents and/or the textile material substantially do not comprise silver and/or any other metals.

**7.** The hygiene product according to any one of the preceding claims, wherein the one or more antimicrobial agents are one or more selected from the group consisting of polyglucosamine (chitosan), arginine, polydiallyldimethylammonium chloride (polyDADMAC), and any combination thereof, preferably polydiallyldimethylammonium chloride (polyDADMAC).

**8.** The hygiene product of any one of the preceding claims, wherein one or more or preferably all of the crosslinkers or binders and/or of the antimicrobial agents are permanently adhered to the textile material.

**9.** The hygiene product of any one of the preceding claims, wherein the textile material comprises at least one acid selected from the group consisting of acetic acid, citric acid, salicylic acid, benzoic acid and sorbic acid, or salts thereof, in particular sodium salts thereof, preferably citric or acetic acid, most preferably citric acid.

**10.** The hygiene product of any one of the preceding claims, wherein the textile material comprised by the first and/or second layers of the hygiene product are obtainable by a method comprising the steps of

- treating the textile material using a liquor application process, preferably a padding process,
- preferably heat-treating the treated textile material at a temperature of at least 80 °C, preferably at least 100 °C, more preferably at least 110 °C, and most preferably at least about 120 °C and/or at a heat-treatment temperature of at most 160 °C, preferably at most 140 °C, more preferably at most 130 °C, and most preferably at most about 120 °C,

- wherein heat-treatment is preferably conducted for a time period of at least 60 seconds, preferably at least 90 seconds, more preferably at least 120 seconds, even more preferably at least 180 seconds, and most preferably for at least 240 seconds and/or for a heat treatment time of at most 600 seconds, preferably at most 500 seconds, more preferably at most 400 seconds, even more preferably at most 300 seconds, and most preferably at most 240 seconds.

**11.** The hygiene product of the preceding claims, wherein the hygiene product is a diaper, adult incontinence product, bed pad, underwear liner, or a sanitary napkin.

**12.** The hygiene product of any one of the preceding claims, comprising an additional layer of absorber material (3) comprising one or more of superabsorbent polymers (SAPs).

**13.** The hygiene product of any one of the preceding claims, comprising a water-impermeable layer (4).

**14.** Method of reducing malodors of urine with a hygiene product according to any one of the preceding claims, comprising bringing the urine in contact with the textile material of the first and/or second layer of the hygiene product.

**15.** Method according to the preceding claim, wherein the urine is passed through the first and/or second layers of the hygiene product.

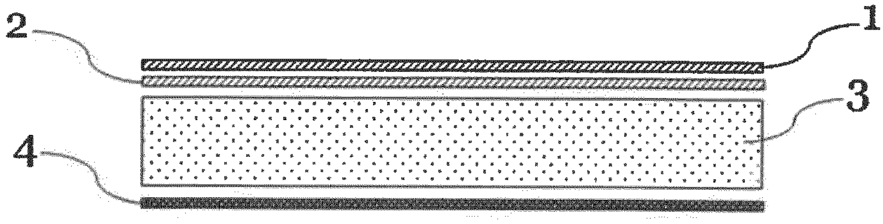


FIG. 1

Europäisches Patentamt
European Patent Office
Office européen des brevets

# EUROPEAN SEARCH REPORT

Application Number
EP 20 16 2736

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 187 047 A1 (GREEN IMPACT HOLDING AG [CH]) 5 July 2017 (2017-07-05)<br>* paragraphs [0234] - [0237], [0240], [0268], [0286] *<br>* paragraphs [0297] - [0308], [0313] - [0317]; claims; examples *<br>----- | 1-15 | INV.<br>A61L15/46<br>A61L15/60 |
| A | WO 2010/009471 A2 (QUICK MED TECHNOLOGIES INC [US]; TOREKI WILLIAM [US] ET AL.) 21 January 2010 (2010-01-21)<br>* the whole document *<br>----- | 1-15 | |
| A | JEON YOU-JIN ET AL: "Effect of antimicrobial activity by chitosan oligosaccharide N-conjugated with asparagine",<br>JOURNAL OF MICROBIOLOGY AND BIOTECHNOLOGY, KOREAN SOCIETY FOR APPLIED MICROBIOLOGY, SEOUL, KR,<br>vol. 11, no. 2, 1 April 2001 (2001-04-01), pages 281-286, XP008081226,<br>ISSN: 1017-7825<br>* the whole document *<br>----- | 1-15 | TECHNICAL FIELDS SEARCHED (IPC)<br>A61L |

The present search report has been drawn up for all claims

1

| Place of search | Date of completion of the search | Examiner |
|---|---|---|
| The Hague | 10 September 2020 | Derrien, Anne-Cécile |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 20 16 2736

This annex lists the patent family members relating to the patent documents cited in the above-mentioned European search report.
The members are as contained in the European Patent Office EDP file on
The European Patent Office is in no way liable for these particulars which are merely given for the purpose of information.

10-09-2020

| Patent document cited in search report | Publication date | Patent family member(s) | Publication date |
|---|---|---|---|
| EP 3187047 A1 | 05-07-2017 | AR 107279 A1 | 18-04-2018 |
| | | AR 107280 A1 | 18-04-2018 |
| | | AU 2017204681 A1 | 16-08-2018 |
| | | BR 112018013517 A2 | 11-12-2018 |
| | | BR 112018013553 A2 | 15-01-2019 |
| | | BR 112018013559 A2 | 11-12-2018 |
| | | CA 3009949 A1 | 06-07-2017 |
| | | CL 2018001740 A1 | 05-10-2018 |
| | | CN 108884626 A | 23-11-2018 |
| | | CN 109310556 A | 05-02-2019 |
| | | CO 2018006891 A2 | 19-07-2018 |
| | | CO 2018006900 A2 | 19-07-2018 |
| | | CO 2018006925 A2 | 20-09-2018 |
| | | EA 201891538 A1 | 31-01-2019 |
| | | EP 3187047 A1 | 05-07-2017 |
| | | EP 3187654 A1 | 05-07-2017 |
| | | EP 3397228 A2 | 07-11-2018 |
| | | EP 3397803 A1 | 07-11-2018 |
| | | HK 1255134 A1 | 09-08-2019 |
| | | HK 1255140 A1 | 09-08-2019 |
| | | JP 2019505696 A | 28-02-2019 |
| | | KR 20180108620 A | 04-10-2018 |
| | | PE 20181469 A1 | 13-09-2018 |
| | | PE 20181470 A1 | 13-09-2018 |
| | | PE 20181471 A1 | 13-09-2018 |
| | | PH 12018501400 A1 | 25-03-2019 |
| | | SG 11201805210W A | 30-07-2018 |
| | | TW 201727006 A | 01-08-2017 |
| | | WO 2017114585 A1 | 06-07-2017 |
| | | WO 2017114971 A2 | 06-07-2017 |
| | | WO 2017114974 A2 | 06-07-2017 |
| WO 2010009471 A2 | 21-01-2010 | AU 2009270715 A1 | 21-01-2010 |
| | | BR PI0911004 A2 | 04-08-2015 |
| | | CA 2731072 A1 | 21-01-2010 |
| | | CN 102131527 A | 20-07-2011 |
| | | EP 2320958 A2 | 18-05-2011 |
| | | US 2010291169 A1 | 18-11-2010 |
| | | WO 2010009471 A2 | 21-01-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **MODOLO et al.** *Journal of Advanced Research,* 2018, vol. 13, 29-37